(19) Europäisches Patentamt
European Patent Office
Office européen des brevets

(11) EP 1 323 429 A1

(12) EUROPEAN PATENT APPLICATION
published in accordance with Art. 158(3) EPC

(43) Date of publication:
**02.07.2003 Bulletin 2003/27**

(21) Application number: **01972487.1**

(22) Date of filing: **26.09.2001**

(51) Int Cl.7: **A61K 45/00**, A61K 31/4985, A61K 31/551, A61K 31/4375, A61K 31/553, A61K 31/4353, A61P 43/00, A61P 25/14, A61P 13/02, A61P 25/24, A61P 1/00, A61P 25/20, C07D 471/14, C07D 498/04

(86) International application number:
**PCT/JP01/08346**

(87) International publication number:
**WO 02/026260 (04.04.2002 Gazette 2002/12)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **26.09.2000 JP 2000297086**
**22.12.2000 JP 2000391037**
**22.12.2000 JP 2000391088**
**22.12.2000 JP 2000391106**

(71) Applicant: **Takeda Chemical Industries, Ltd.
Osaka-shi, Osaka 540-8645 (JP)**

(72) Inventors:
- **DOI, Takayuki
Osaka-shi, Osaka 545-0042 (JP)**
- **BAN, Toshikazu
Ikoma-shi, Nara 630-0213 (JP)**

(74) Representative:
**von Kreisler, Alek, Dipl.-Chem. et al
Patentanwälte
von Kreisler-Selting-Werner
Postfach 10 22 41
50462 Köln (DE)**

# (54) PREVENTIVES/REMEDIES FOR EMOTIONAL DISORDERS

(57) An agent for the prophylaxis or treatment of an emotional disorder, which contains the NK-1 receptor antagonist having particular properties of (1) having no serotonin uptake inhibitory effect, (2) being capable of migrating into the hypothalamus, or (3) having an inhibitory effect on micturition reflex, an agent for the prophylaxis or treatment of depression accompanied by urinary frequency, urinary incontinence and/or irritable bowel syndrome, which contains an NK-1 receptor antagonist, an agent for the prophylaxis or treatment of a mood disorder of patients with urinary frequency and urinary incontinence, and a circadian rhythm controller for the hypothalamic endocrine system are provided.

EP 1 323 429 A1

## Description

### Technical Field

The present invention relates to an agent for the prophylaxis or treatment of an emotional disorder, particularly an agent for the prophylaxis or treatment of depression or a mood disorder, which contains an NK-1 receptor antagonist having particular property, a circadian rhythm controller for the hypothalamic endocrine system, and a screening method of an NK-1 receptor antagonist having such particular property. Moreover, the present invention relates to novel pharmaceutical use of an NK-1 receptor antagonist.

### Background Art

It has been reported that an NK-1 receptor antagonist is effective for the treatment of depression (WO98/15277, WO98/24438, WO98/24441), anxiety (WO98/15277, WO98/24469), consciousness disorder (WO98/24447), schizophrenia (WO98/2445) and the like. However, the above-mentioned conventionally known compounds have been reported to show a side effect such as hypogonadism and the like, whereas a pharmaceutical agent, which is capable of preventing or treating an emotional disorder, particularly depression, a mood disorder, abnormal circadian rhythm of the hypothalamic endocrine system, and the like, and which shows remarkably reduced side effect, has not been reported.

### Disclosure of the Invention

The present invention aims at solving the above-mentioned problems and providing a pharmaceutical agent, which is capable of preventing or treating an emotional disorder, particularly depression, a mood disorder, abnormal circadian rhythm of the hypothalamic endocrine system, and the like, and which shows remarkably reduced side effects.

As a result of the intensive studies in view of the above-mentioned situation, the present inventors have found that an NK-1 receptor antagonist which has specific characteristics of (1) having no serotonin uptake inhibitory effect, (2) being capable of migrating into the hypothalamus, or (3) having an inhibitory effect on micturition reflex, from among various NK-1 receptor antagonists, is unexpectedly free of side effects such as hypogonadism and the like and can be a clinically extremely useful agent for the prophylaxis or treatment of an emotional disorder, particularly a pharmaceutical agent useful for depression, a mood disorder and abnormal circadian rhythm of the hypothalamic endocrine system, and further found novel use of an NK-1 receptor antagonist as an agent for the prophylaxis or treatment of depression accompanied by urinary frequency, urinary incontinence and/or irritable bowel syndrome, an agent for the prophylaxis or treatment of mood disorders of patients with urinary frequency and urinary incontinence, and a circadian rhythm controller for the hypothalamic endocrine system, which resulted in the completion of the present invention.

Accordingly, the present invention provides

[1] an agent for the prophylaxis or treatment of an emotional disorder which comprises an NK-1 receptor antagonist having at least one of the following properties:

(i) having no serotonin uptake inhibitory effect,
(ii) being capable of migrating into the hypothalamus,
(iii) having an inhibitory effect on micturition reflex,

[2] the agent for the prophylaxis or treatment for an emotional disorder of the above-mentioned [1], wherein the emotional disorder is accompanied by depression, depression accompanied by urinary frequency, urinary incontinence and/or irritable bowel syndrome, a mood disorder (particularly a mood disorder of patients with urinary frequency and urinary incontinence), or abnormal circadian rhythm of the hypothalamic endocrine system,

[3] the agent for the prophylaxis or treatment of an emotional disorder of the above-mentioned [1] or [2], which is used for oral administration,

[4] (i) an agent for the prophylaxis or treatment of depression accompanied by urinary frequency, urinary incontinence and/or irritable bowel syndrome, (ii) an agent for the prophylaxis or treatment of a mood disorder of patients with urinary frequency and urinary incontinence, or (iii) a circadian rhythm controller for the hypothalamic endocrine system, which comprises an NK-1 receptor antagonist,

[5] the various pharmaceutical agents of the above-mentioned [4], which are used for oral administration,

[6] the various pharmaceutical agents of the above-mentioned [4], wherein the NK-1 receptor antagonist has at least one of the following properties:

(i) having no serotonin uptake inhibitory effect,
(ii) being capable of migrating into the hypothalamus,
(iii) having an inhibitory effect on micturition reflex,

[7] a pharmaceutical composition for the prophylaxis or treatment of an emotional disorder, which comprises an NK-1 receptor antagonist having at least one of the following properties and a pharmaceutically acceptable carrier:

(i) having no serotonin uptake inhibitory effect,
(ii) being capable of migrating into the hypothalamus,
(iii) having an inhibitory effect on micturition reflex,

[8] the pharmaceutical composition for the prophylaxis or treatment of an emotional disorder of the above-mentioned [7], wherein the emotional disorder is accompanied by depression, depression accompanied by urinary frequency, urinary incontinence and/or irritable bowel syndrome, a mood disorder (particularly a mood disorder of patients with urinary frequency and urinary incontinence) or abnormal circadian rhythm of the hypothalamic endocrine system,
[9] (i) a pharmaceutical composition for the prophylaxis or treatment of depression accompanied by urinary frequency, urinary incontinence and/or irritable bowel syndrome, (ii) a pharmaceutical composition for the prophylaxis or treatment of a mood disorder of patients with urinary frequency and urinary incontinence, or (iii) a pharmaceutical composition for controlling circadian rhythm of the hypothalamic endocrine system, which comprises an NK-1 receptor antagonist and a pharmaceutically acceptable carrier,
[10] the various pharmaceutical compositions of the above-mentioned [9], wherein the NK-1 receptor antagonist has at least one of the following properties:

(i) having no serotonin uptake inhibitory effect,
(ii) being capable of migrating into the hypothalamus,
(iii) having an inhibitory effect on micturition reflex,

[11] a method for the prophylaxis or treatment of an emotional disorder, which comprises administering an effective amount of an NK-1 receptor antagonist having at least one of the following properties to patients:

(i) having no serotonin uptake inhibitory effect,
(ii) being capable of migrating into the hypothalamus,
(iii) having an inhibitory effect on micturition reflex,

[12] the method of the above-mentioned [11], wherein the emotional disorder is accompanied by depression, depression accompanied by urinary frequency, urinary incontinence and/or irritable bowel syndrome, a mood disorder (particularly a mood disorder of patients with urinary frequency and urinary incontinence) or abnormal circadian rhythm of the hypothalamic endocrine system,
[13] (i) a method for the prophylaxis or treatment of depression accompanied by urinary frequency, urinary incontinence and/or irritable bowel syndrome, (ii) a method for the prophylaxis or treatment of a mood disorder of patients with urinary frequency and urinary incontinence, or (iii) a method for controlling circadian rhythm of the hypothalamic endocrine system of patients, which comprises administering an effective amount of an NK-1 receptor antagonist to patients,
[14] the method of the above-mentioned [13], wherein the NK-1 receptor antagonist has at least one of the following properties:

(i) having no serotonin uptake inhibitory effect,
(ii) being capable of migrating into the hypothalamus,
(iii) having an inhibitory effect on micturition reflex,

[15] use of an NK-1 receptor antagonist for the production of an agent for the prophylaxis or treatment of an emotional disorder, wherein the antagonist has at least one of the following properties:

(i) having no serotonin uptake inhibitory effect,
(ii) being capable of migrating into the hypothalamus,
(iii) having an inhibitory effect on micturition reflex,

[16] the use of the above-mentioned [15], wherein the emotional disorder is accompanied by depression, depression accompanied by urinary frequency, urinary incontinence and/or irritable bowel syndrome, a mood disorder (particularly a mood disorder of patients with urinary frequency and urinary incontinence) or abnormal circadian rhythm of the hypothalamic endocrine system,
[17] use of an NK-1 receptor antagonist for the production of (i) an agent for the prophylaxis or treatment of depression accompanied by urinary frequency, urinary incontinence and/or irritable bowel syndrome, (ii) an agent for the prophylaxis or treatment of a mood disorder of patients with urinary frequency and urinary incontinence, or (iii) circadian rhythm controllers for the hypothalamic endocrine system,
[18] the use of the above-mentioned [17], wherein the NK-1 receptor antagonist has at least one of the following properties:

(i) having no serotonin uptake inhibitory effect,
(ii) being capable of migrating into the hypothalamus,
(iii) having an inhibitory effect on micturition reflex,

[19] a commercial package comprising the pharmaceutical composition of the above-mentioned [7] and written matter associated therewith, the written matter stating that the pharmaceutical composition can or should be used for the prophylaxis or treatment of an emotional disorder,
[20] the commercial package of the above-mentioned [19], wherein the emotional disorder is accompanied by depression, depression accompanied by urinary frequency, urinary incontinence and/or irritable bowel syndrome, a mood disorder (particularly a mood disorder of patients with urinary frequency and urinary incontinence) or abnormal circadian rhythm of the hypothalamic endocrine system,
[21] a commercial package comprising the pharmaceutical composition of the above-mentioned [8] and written matter associated therewith, the written matter stating that the pharmaceutical composition can or should be used (i) for the prophylaxis or treatment of depression accompanied by urinary frequency, urinary incontinence and/or irritable bowel syndrome, (ii) for the prophylaxis or treatment of a mood disorder of patients with urinary frequency and urinary incontinence or (iii) for controlling circadian rhythm of the hypothalamic endocrine system,
[22] a screening method of an NK-1 receptor antagonist, which comprises measuring an NK-1 receptor antagonist group for at least one action selected from the group consisting of (i) a serotonin uptake inhibitory effect, (ii) capability of migration into the hypothalamus and (iii) an inhibitory effect on micturition reflex, and determining an NK-1 receptor antagonist having at least one of the following properties:

(i) having no serotonin uptake inhibitory effect,
(ii) being capable of migrating into the hypothalamus,
(iii) having an inhibitory effect on micturition reflex of not less than 25% as compared to a control group, and

[23] various pharmaceutical agents of any of the above-mentioned [1] to [6], which comprises an NK-1 receptor antagonist obtained by the screening method of the above-mentioned [22].

The present invention further provides
[24] a pharmaceutical agent of the above-mentioned each item, wherein the NK-1 receptor antagonist or the NK-1 receptor antagonist having particular properties (the aforementioned) is a compound (I) represented by the formula

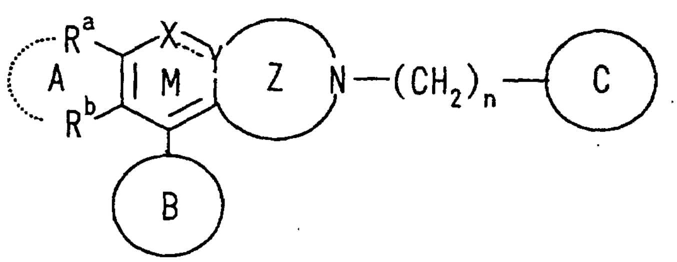

wherein Ring M is a heterocyclic ring wherein

$$-X\overline{\text{----------}}Y<$$

is one of -N=C<, -CO-N< and -CS-N<;
$R^a$ and $R^b$ are bonded to each other to form Ring A, or the same or different and each represents a hydrogen atom or a substituent on the Ring M;
Ring A and Ring B each represent an optionally substituted homocyclic ring or heterocyclic ring, with the proviso that at least one of them is an optionally substituted heterocyclic ring;
Ring C is an optionally substituted homocyclic ring or heterocyclic ring;
Ring Z is an optionally substituted nitrogen-containing heterocyclic ring; and
n is an integer from 1 to 6, or a salt thereof or a prodrug thereof,
[25] The pharmaceutical agent as defined in the above-mentioned [24], wherein $R^a$ and $R^b$ each represent a hydrogen. atom or a substituent on the Ring M selected from the group consisting of

(1) a halogen atom,
(2) a $C_{1-6}$ alkyl group optionally having from 1 to 5 substituents selected from the group consisting of

(a) a hydroxy group,
(b) a $C_{1-6}$ alkoxy group,
(c) a $C_{1-6}$ alkylthio group,
(d) an amino group,
(e) a $C_{1-7}$ acylamino group,
(f) a carboxyl group,
(g) a nitro group,
(h) a mono- or di-$C_{1-6}$ alkylamino group,
(i) a mono- or di-$C_{3-8}$ cycloalkylamino group,
(j) a $C_{6-10}$ arylamino group,
(k) a 5-membered to 9-membered cyclic amino group which may have 1 to 3 hetero atoms selected from the group consisting of oxygen and sulfur atoms in addition to nitrogen atom and which may be substituted by $C_{1-6}$ alkyl group,
(l) a 5-membered or 6-membered aromatic heterocyclic group having from 1 to 3 hetero atoms selected from the group consisting of nitrogen, oxygen and sulfur atoms in addition to carbon atoms,
(m) a 5-membered to 9-membered non-aromatic heterocyclic group having from 1 to 3 hetero atoms selected from the group consisting of nitrogen, oxygen and sulfur atoms in addition to carbon atoms,
(n) a $C_{1-4}$ alkylsulfonylamino group,
(o) a $C_{1-6}$ alkyl-carbonyloxy group and
(p) a halogen atom,

(3) an optionally halogenated $C_{1-6}$ alkoxy group,
(4) an optionally halogenated $C_{1-6}$ alkylthio group,
(5) a $C_{3-10}$ cycloalkyl group,
(6) a $C_{6-10}$ aryl group,
(7) a $C_{1-7}$ acylamino group,
(8) a $C_{1-3}$ acyloxy group,
(9) a hydroxy group,
(10) a nitro group,
(11) a cyano group,
(12) an amino group,
(13) a mono- or di-$C_{1-6}$ alkylamino group,
(14) a 5-membered to 9-membered cyclic amino group which may have 1 to 3 hetero atoms selected from the group consisting of oxygen and sulfur atoms in addition to nitrogen atom and which may be substituted by $C_{1-6}$ alkyl group,
(15) a $C_{1-6}$ alkyl-carbonylamino group,
(16) a $C_{1-6}$ alkyl-sulfonylamino group,
(17) a $C_{1-6}$ alkoxycarbonyl group,
(18) a carboxyl group,
(19) a $C_{1-6}$ alkylcarbonyl group,
(20) a carbamoyl group,
(21) a mono- or di-$C_{1-6}$ alkylcarbamoyl group and
(22) a $C_{1-6}$ alkylsulfonyl group and
(23) an oxo group, or

$R^a$ and $R^b$ are bonded to each other to form Ring A, and the Ring A is

(i) a 5-membered to 9-membered aromatic heterocyclic ring having 1 to 3 of hetero atoms of the same type or two different types selected from the group consisting of nitrogen, sulfur and oxygen atoms in addition to carbon atoms,
(ii) a 5-membered to 9-membered non-aromatic heterocyclic ring having 1 to 3 hetero atoms of the same type or two different types selected from the group consisting of nitrogen, sulfur and oxygen atoms in addition to carbon atoms, or
(iii) a 3-membered to 10-membered cyclic hydrocarbon each of which may have 1 to 4 substituents selected from

(1) a halogen atom,
(2) a $C_{1-6}$ alkyl group optionally having from 1 to 5 substituents selected from the group consisting of

(a) a hydroxy group,
(b) an amino group,
(c) a carboxyl group,
(d) a nitro group,
(e) a mono- or di-$C_{1-6}$ alkylamino group,
(f) a $C_{1-6}$ alkyl-carbonyloxy group and
(g) a halogen atom,

(3) an optionally halogenated $C_{1-6}$ alkoxy group,
(4) an optionally halogenated $C_{1-6}$ alkylthio group,
(5) a $C_{6-10}$ aryl group,
(6) a $C_{1-7}$ acylamino group,
(7) a $C_{1-3}$ acyloxy group,
(8) a hydroxy group,
(9) a nitro group,
(10) a cyano group,
(11) an amino group,
(12) a mono- or di-$C_{1-6}$ alkylamino group,
(13) a 5-membered to 9-membered cyclic amino group which may have 1 to 3 hetero atoms selected from the group consisting of oxygen and sulfur atoms in addition to nitrogen atom,
(14) a $C_{1-6}$ alkyl-carbonylamino group,
(15) a $C_{1-6}$ alkyl-sulfonylamino group,
(16) a $C_{1-6}$ alkoxy-carbonyl group,
(17) a carboxyl group,
(18) a $C_{1-6}$ alkylcarbonyl group,
(19) a carbamoyl group,
(20) a mono- or di-$C_{1-6}$ alkylcarbamoyl group,
(21) a $C_{1-6}$ alkylsulfonyl group, or
(22) an oxo group;

the Ring B is a

(i) 5-membered to 9-membered aromatic heterocyclic ring having 1 to 3 hetero atoms of the same type or two different types selected from the group consisting of nitrogen, sulfur and oxygen atoms in addition to carbon atoms,
(ii) a 5-membered to 9-membered non-aromatic heterocyclic ring having 1 to 3 hetero atoms of the same type or two different types selected from the group consisting of nitrogen, sulfur and oxygen atoms in addition to carbon atoms, or
(iii) a 3-membered to 10-membered cyclic hydrocarbon each of which may have 1 to 4 substituents selected from the group consisting of

(1) a halogen atom,
(2) a $C_{1-6}$ alkyl group optionally having from 1 to 5 substituents selected from the group consisting of

(a) a hydroxy group,

(b) an amino group,
(c) a carboxyl group,
(d) a nitro group,
(e) a mono- or di-$C_{1-6}$ alkylamino group,
(f) a $C_{1-6}$ alkyl-carbonyloxy group and
(g) a halogen atom,

(3) an optionally halogenated $C_{1-6}$ alkoxy group,
(4) an optionally halogenated $C_{1-6}$ alkylthio group,
(5) a $C_{6-10}$ aryl group,
(6) a $C_{1-7}$ acylamino group,
(7) a $C_{1-3}$ acyloxy group,
(8) a hydroxy group,
(9) a nitro group,
(10) a cyano group,
(11) an amino group,
(12) a mono- or di-$C_{1-6}$ alkylamino group,
(13) a 5-membered to 9-membered cyclic amino group which may have 1 to 3 hetero atoms selected from the group consisting of oxygen and sulfur atoms in addition to nitrogen atom,
(14) a $C_{1-6}$ alkyl-carbonylamino group,
(15) a $C_{1-6}$ alkyl-sulfonylamino group,
(16) a $C_{1-6}$ alkoxy-carbonyl group,
(17) a carboxyl group,
(18) a $C_{1-6}$ alkylcarbonyl group,
(19) a carbamoyl group,
(20) a mono- or di-$C_{1-6}$ alkylcarbamoyl group,
(21) a $C_{1-6}$ alkylsulfonyl group, and
(22) an oxo group;
the Ring C is

(i) a 5-membered to 9-membered heterocyclic ring which may have 1 to 3 hetero atoms of the same type or two different types selected from the group consisting of nitrogen, sulfur and oxygen atoms in addition to carbon atoms, which optionally having 1 to 5 substituents selected from the group consisting of

(1) a halogen atom,
(2) an optionally halogenated $C_{1-10}$ alkyl group,
(3) an amino-substituted $C_{1-4}$ alkyl group,
(4) a mono- or di-$C_{1-4}$ alkylamino-substituted $C_{1-4}$ alkyl group,
(5) a carboxyl-substituted $C_{1-4}$ alkyl group,
(6) a $C_{1-4}$ alkoxy-carbonyl-substituted $C_{1-4}$ alkyl group,
(7) a hydroxy-substituted $C_{1-4}$ alkyl group,
(8) a $C_{3-10}$ cycloalkyl group,
(9) a nitro group,
(10) a cyano group,
(11) a hydroxy group,
(12) an optionally halogenated $C_{1-10}$ alkoxy group,
(13) an optionally halogenated $C_{1-4}$ alkylthio group,
(14) an amino group,
(15) a mono- or di-$C_{1-4}$ alkylamino group,
(16) a 5-membered to 9-membered cyclic amino group optionally having 1 to 3 hetero atoms selected from the group consisting of oxygen and sulfur atoms in addition to nitrogen atom,
(17) a $C_{1-4}$ alkyl-carbonylamino group,
(18) an aminocarbonyloxy group,
(19) a mono- or di-$C_{1-4}$ alkylaminocarbonyloxy group,
(20) a $C_{1-4}$ alkylsulfonylamino group,
(21) a $C_{1-4}$ alkoxy-carbonyl group,
(22) an aralkyloxycarbonyl group,
(23) a carboxyl group,

(24) a $C_{1-6}$ alkyl-carbonyl group,
(25) a $C_{3-6}$ cycloalkyl-carbonyl group,
(26) a carbamoyl group,
(27) a mono- or di-$C_{1-4}$ alkylcarbamoyl group,
(28) a $C_{1-3}$ acyloxy group,
(29) a $C_{1-6}$ alkylsulfonyl group and
(30) a 5-membered or 6-membered aromatic monocyclic heterocyclic ring having 1 to 4 hetero atoms selected from the group consisting of nitrogen, sulfur and oxygen atoms in addition to carbon atoms, which may be substituted by 1 to 3 optionally halogenated $C_{1-4}$ alkyls;, or

(ii) a 3-membered to 10-membered homocyclic ring, optionally having 1 to 5 substituents selected from the group consisting of

(1) a halogen atom,
(2) an optionally halogenated $C_{1-10}$ alkyl group,
(3) an amino-substituted $C_{1-4}$ alkyl group,
(4) a mono- or di-$C_{1-4}$ alkylamino-substituted $C_{1-4}$ alkyl group,
(5) a carboxyl-substituted $C_{1-4}$ alkyl group,
(6) a $C_{1-4}$ alkoxy-carbonyl-substituted $C_{1-4}$ alkyl group,
(7) a hydroxy-substituted $C_{1-4}$ alkyl group,
(8) a $C_{3-10}$ cycloalkyl group,
(9) a nitro group,
(10) a cyano group,
(11) a hydroxy group,
(12) an optionally halogenated $C_{1-10}$ alkoxy group,
(13) an optionally halogenated $C_{1-4}$ alkylthio group,
(14) an amino group,
(15) a mono- or di-$C_{1-4}$ alkylamino group,
(16) a 5-membered to 9-membered cyclic amino group optionally having 1 to 3 hetero atoms selected from the group consisting of oxygen and sulfur atoms in addition to nitrogen atom,
(17) a $C_{1-4}$ alkyl-carbonylamino group,
(18) an aminocarbonyloxy group,
(19) a mono- or di-$C_{1-4}$ alkylaminocarbonyloxy group,
(20) a $C_{1-4}$ alkylsulfonylamino group,
(21) a $C_{1-4}$ alkoxy-carbonyl group,
(22) an aralkyloxycarbonyl group,
(23) a carboxyl group,
(24) a $C_{1-6}$ alkylcarbonyl group,
(25) a $C_{3-6}$ cycloalkyl-carbonyl group,
(26) a carbamoyl group,
(27) a mono- or di-$C_{1-4}$ alkylcarbamoyl group,
(28) a $C_{1-3}$ acyloxy group,
(29) a $C_{1-6}$ alkylsulfonyl group and
(30) a 5-membered or 6-membered aromatic monocyclic heterocyclic ring having 1 to 4 hetero atoms selected from the group consisting of nitrogen, sulfur and oxygen atoms in addition to carbon atoms, which may be substituted by 1 to 3 optionally halogenated $C_{1-4}$ alkyls;

the Ring Z is a 5-membered to 12-membered heterocyclic ring optionally having at least one hetero atom selected from the group consisting of nitrogen, oxygen and sulfur atoms in addition to Y, carbon atoms and nitrogen atom, and optionally having 1 to 5 substituents selected from the group consisting of

(1) a $C_{1-6}$ alkyl group,
(2) a $C_{2-6}$ alkenyl group,
(3) a $C_{2-6}$ alkynyl group,
(4) a $C_{3-8}$ cycloalkyl group,
(5) a $C_{3-8}$ cycloalkyl-$C_{1-4}$ alkyl group,
(6) a $C_{6-14}$ aryl group,
(7) a nitro group,

(8) a cyano group,
(9) a hydroxy group,
(10) a $C_{1-4}$ alkoxy group,
(11) a $C_{1-4}$ alkylthio group,
(12) a amino group,
(13) a mono- or di-$C_{1-4}$ alkylamino group,
(14) a 5-membered to 9-membered cyclic amino group optionally having 1 to 3 hetero atoms selected from the group consisting of oxygen and sulfur atoms in addition to nitrogen atom,
(15) a $C_{1-4}$ alkyl-carbonylamino group,
(16) a $C_{1-4}$ alkylsulfonylamino group,
(17) a $C_{1-4}$ alkoxy-carbonyl group,
(18) a carboxyl group,
(19) a $C_{1-6}$ alkyl-carbonyl group,
(20) a carbamoyl group,
(21) a mono- or di-$C_{1-4}$ alkylcarbamoyl group,
(22) a $C_{1-6}$ alkylsulfonyl group,
(23) an oxo group, and
(24) a thioxo group;

[26] The pharmaceutical agent of the above-mentioned [25], wherein $R^a$ and $R^b$ are bonded together to form Ring A, Ring C is a benzene ring optionally having substituents or heterocyclic ring optionally having substituents, Ring Z is an optionally oxoated nitrogen-containing heterocyclic ring, and n is 1 or 2,

[27] The pharmaceutical agent of the above-mentioned [25], wherein the Ring Z is an optionally oxoated nitrogen-containing heterocyclic ring,

[28] The pharmaceutical agent of the above-mentioned [25], wherein one of the Ring A and the Ring B is an aromatic homocyclic ring optionally having substituents and the other is an aromatic heterocyclic ring optionally having substituents,

[29] the pharmaceutical agent of the above-mentioned [25], wherein the Ring A is an aromatic heterocyclic ring optionally having substituents and the Ring B is a benzene ring optionally having substituents,

[30] the pharmaceutical agent of the above-mentioned [29], wherein the aromatic heterocyclic ring is a 5-membered or 6-membered aromatic heterocyclic ring containing one or two kinds of hetero atoms selected from nitrogen, sulfur and oxygen atoms in addition to carbon atoms,

[31] the pharmaceutical agent of the above-mentioned [25], wherein the Ring C is an optionally substituted benzene ring,

[32] the pharmaceutical agent of the above-mentioned [25], wherein the Ring C is a benzene ring having 1 to 3 substituents selected from halogen atom, optionally halogenated $C_{1-6}$ alkyl group and optionally halogenated $C_{1-6}$ alkoxy group,

[33] the pharmaceutical agent of the above-mentioned [25], wherein the Ring Z is a 5-membered to 10-membered heterocyclic ring optionally substituted by 1 or 2 oxo groups,

[34] the pharmaceutical agent of the above-mentioned [25], wherein

$$—X\overline{\text{----------}}Y< \text{ is } -CO-N< \text{ or } -N=C<,$$

[35] the pharmaceutical agent of the above-mentioned [25], wherein n is 1,

[36] the pharmaceutical agent of the above-mentioned [25], wherein Ring A is a pyridine ring optionally having substituents, Ring B is a benzene ring optionally having substituents, Ring C is a benzene ring optionally having substituents, Ring Z is an optionally oxoated 5-membered to 10-membered heterocyclic ring,

$$—X\overline{\text{----------}}Y< \text{ is } -CO-N< \text{ or } -N=C<;$$

and
n is 1,

[37] the pharmaceutical agent of the above-mentioned [25], wherein $R^a$ and $R^b$ are the same or different and each is a hydrogen atom, a halogen atom, an alkyl group optionally having substituents, an optionally halogenated alkoxy group, an optionally halogenated alkylthio group, a cycloalkyl group, an aryl group, an acylamino group, an acyloxy group, a hydroxyl group, a nitro group, a cyano group, an amino group, a mono- or di-alkylamino group, a cyclic amino group, an alkylcarbonylamino group, an alkylsulfonylamino group, an alkoxycarbonyl group, a carboxyl group, an alkylcarbonyl group, a carbamoyl group, a mono- or di-alkylcarbamoyl group, an alkylsulfonyl group or an oxo group,

[38] the pharmaceutical agent of the above-mentioned [25], wherein $R^a$ and $R^b$ are the same or different and each is a hydrogen atom or a $C_{1-6}$ alkyl group optionally substituted by (i) a $C_{1-6}$ alkoxy group, (ii) a $C_{1-6}$ alkylthio group, (iii) an amino group, (iv) a $C_{1-7}$ acylamino group, (v) a mono- or di-$C_{1-6}$ alkylamino group, (vi) a $C_{5-9}$ cyclic amino group (5-membered to 9-membered cyclic amino group optionally containing 1 to 3 hetero atoms such as oxygen atom, sulfur atom and the like, in addition to nitrogen atom), (vii) a 5-membered or 6-membered cyclic amino group optionally substituted by $C_{1-6}$ alkyl group, (viii) a $C_{1-6}$ alkylsulfonylamino group or (ix) a $C_{1-6}$ alkylcarbonyloxy group, or $R^a$ and $R^b$ are bonded to form a pyridine ring optionally having 1 to 3 substituent(s) selected from halogen atom and $C_{1-4}$ alkyl group,

Ring B is a benzene ring optionally having 1 to 3 substituent(s) selected from (i) a halogen atom, (ii) an optionally halogenated $C_{1-4}$ alkyl group and (iii) an optionally halogenated $C_{1-4}$ alkoxy group,

Ring C is a benzene ring optionally having 1 to 3 substituent(s) selected from (i) a halogen atom, (ii) an optionally halogenated $C_{1-4}$ alkyl group, (iii) an optionally halogenated $C_{1-4}$ alkoxy group, (iv) an amino group optionally substituted by $C_{1-4}$ alkyl group, (v) a $C_{1-3}$ acyloxy group and (vi) a hydroxyl group,

Ring Z is a 5-membered to 10-membered nitrogen-containing heterocyclic group, which is optionally substituted by a $C_{1-4}$ alkyl group or a hydroxyl group and which is optionally oxoated,

— X $\overline{\text{----------}}$ Y< is -CO-N< or -N=C< ;

and

n is 1,

[39] the pharmaceutical agent of the above-mentioned [38], wherein $R^a$ and $R^b$ are bonded to form a pyridine ring optionally having 1 to 3 substituent(s) selected from a halogen atom and a $C_{1-4}$ alkyl group, and

— X $\overline{\text{----------}}$ Y< is -CO-N< ,

[40] the pharmaceutical agent of the above-mentioned [39], wherein the pyridine ring is an unsubstituted pyridine ring,

[41] the pharmaceutical agent of the above-mentioned [38], wherein the Ring B is a benzene ring optionally having 1 to 3 optionally halogenated $C_{1-4}$ alkyl groups,

[42] the pharmaceutical agent of the above-mentioned [38], wherein the Ring C is a benzene ring optionally having 1 to 3 substituent(s) selected from (i) a halogen atom, (ii) an optionally halogenated $C_{1-4}$ alkyl group and (iii) an optionally halogenated $C_{1-4}$ alkoxy group,

[43] the pharmaceutical agent of the above-mentioned [38], wherein the Ring Z is represented by the formula

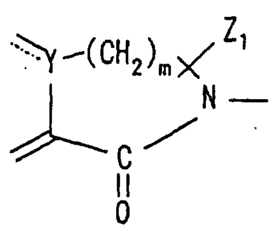

or

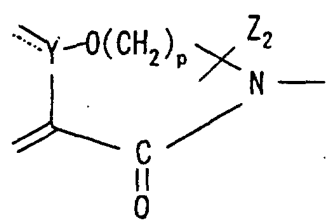

wherein m and p are the same or different and each is an integer of 1 to 5, $Z_1$ and $Z_2$ are the same or different and each is a hydrogen atom, a $C_{1-4}$ alkyl group or a hydroxyl group and Y is C or N, and

[44] the pharmaceutical agent of the above-mentioned [24], wherein the compound (I) is (i) (9S)-7-[3,5-bis(trifluoromethyl)benzyl]-6,7,8,9,10,12-hexahydro-9-methyl-6,12-dioxo-5-phenyl[1,4]diazepino[2,1-g][1,7]naphthyridine, (ii) (9S)-7-[3,5-bis(trifluoromethyl)benzyl]-6,7,8,9,10,12-hexahydro-9-methyl-5-(4-methyl)phenyl-6,12-dioxo[1,4]diazepino[2,1-g][1,7]naphthyridine, (iii) (9R)-7-[3,5-bis(trifluoromethyl)benzyl]-6,7,8,9,10,11-hexahydro-9-methyl-6,13-dioxo-5-phenyl-13H-[1,4]diazocino[2,1-g] [1,7]naphthyridine, (iv) (9R)-7-[3,5-bis(trifluoromethyl)benzyl]-6,7,8,9,10,11-hexahydro-9-methyl-5-(4-methylphenyl)-6,13-dioxo-13H-[1,4]diazocino[2,1-g][1,7]naphthyridine, (v) (9R)-7-(3,5-dimethoxybenzyl)-5-(4-fluorophenyl)-6,7,8,9,10,11-hexahydro-9-methyl-6,13-dioxo-13H-[1,4]diazocino[2,1-g][1,7]naphthyridine or (vi) (9R)-7-(3,5-dimethoxybenzyl)-6,7,8,9,10,11-hexahydro-9-methyl-5-(4-methylphenyl)-6,13-dioxo-13H-[1,4]diazocino[2,1-g][1,7]naphthyridine.

The compound (I) and a salt thereof encompass a compound wherein $R^a$ and $R^b$ are bonded to each other to form Ring A, which is represented by the formula

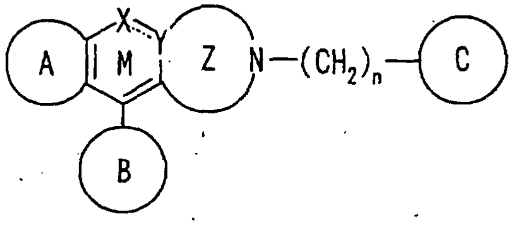

(Ia)

wherein each symbol is as defined above.

The NK-1 receptor antagonist to be used for the prophylaxis or treatment of an emotional disorder of the present invention has one, preferably two, more preferably all three, of the particular properties of

(1) having no serotonin uptake inhibitory effect,
(2) being capable of migrating into the hypothalamus, and
(3) having an inhibitory effect on micturition reflex.

As used herein, by the "emotional disorder" is meant a disease state of a mood disorder and anxiety disorder, whose concrete symptoms are depression, depression accompanied by urinary frequency, urinary incontinence and/or irritable bowel syndrome, a mood disorder (particularly a mood disorder of patients with urinary incontinence) and the like, wherein the emotional disorder may accompany abnormal circadian rhythm of the hypothalamic endocrine system.

Mammals have three kinds of neurokinin receptors of NK-1, NK-2 and NK-3. The NK-1 receptor antagonist means a substance that binds with the NK-1 receptor and inhibits the action of neurokinin. The NK-1 receptor antagonistic action can be measured according to a receptor binding experiment using an NK-1 receptor.

By the "having no serotonin uptake inhibitory effect" is meant that the serotonin uptake inhibitory effect is below detection level.

The serotonin uptake inhibitory effect can be measured according to a method known per se, such as the method described in The Journal of Biological Chemistry, pp. 7124-7130, vol. 269, 1994 or a similar method.

Even if the serotonin uptake inhibitory effect is within the detectable level, an NK-1 receptor antagonist capable of suppressing serotonin uptake by not less than about 25%, preferably not less than about 50%, more preferably not less than about 80%, as compared to commercially available drugs reported to have similar efficacy, can be preferably used in the present invention.

In the present invention, the serotonin "uptake action" encompasses a "re-uptake action" in a narrow sense and both actions are known in this field.

By the "being capable of migrating into the hypothalamus" is meant that an NK-1 receptor antagonist or a prodrug thereof can reach the hypothalamus.

The capability of migration into the hypothalamus can be measured according to a method known per se, such as the method described in Folia pharmacol.japon., pp. 109-123, vol. 88, 1986 or a similar method.

In the present invention, moreover, an NK-1 receptor antagonist capable of migrating into brain tissues other than hypothalamus (e.g., frontal lobe, occipital lobe, thalamus) or a prodrug thereof can be also used.

By the "having an inhibitory effect on micturition reflex" is meant, for example, an inhibitory effect on micturition reflex of not less than 25%, preferably not less than about 50%, more preferably not less than about 80%, as compared to a control group. As used herein, "a control group" means a group to which only a solvent of the drug is administered.

The inhibitory effect on micturition reflex can be measured according to a method known per se, such as the method described in European Journal of Pharmacology, pp. 241-246, vol. 395, 2000 or a similar method.

The NK-1 receptor antagonist having the above-mentioned particular properties shows dramatic reduction of side effects such as hypogonadism (e.g., male erectile dysfunction, function disorder of female genitalia) and the like found in conventional antidepressant and the like, shows low toxicity and is safe. Therefore, a pharmaceutical agent or a pharmaceutical composition containing the NK-1 receptor antagonist having the above-mentioned particular properties is useful as an agent for the prophylaxis or treatment of an emotional disorder, particularly an agent for the prophylaxis or treatment of diseases such as depression, anxiety, manic depression, schizophrenia, depression accompanied by urinary frequency, urinary incontinence and/or irritable bowel syndrome, a mood disorder of patients

with urinary incontinence and urinary frequency, abnormal circadian rhythm of the hypothalamic endocrine system and the like, in mammals (e.g., mouse, rat, hamster, rabbit, cat, dog, bovine, sheep, monkey, human and the like).

The NK-1 receptor antagonist having the above-mentioned particular properties and used as an agent for the prophylaxis or treatment of an emotional disorder in the present invention can be concretely selected from the NK-1 receptor antagonists described in JP-A-9-263585, EP0652218, EP0360390, EP0394989, EP0429366, EP0443132, EP0482539, EP0512901, EP0512902, EP0541273, EP0514275, EP0517589, EP0520555, EP0522808, EP0528495, EP0532456, EP0533280, EP0536817, EP0545478, EP0577394, EP0590152, EP0599538, EP0610793, EP0634402, EP0686629, EP0639489, EP0694535, EP0699655, EP0699674, EP0707006, EP0708101, EP0714891, EP0723959, EP0733632, EP0776893, WO90/05525, WO90/05729, WO91/09844, WO91/18899, WO92/01688, WO92/06079, WO92/12151, WO92/15585, WO92/117449, WO92/20661, WO92/20676, WO92/21677, WO93/00330, WO93/00331, WO93/01159, WO93/01165, WO93/01169, WO93/01170, WO93/06099, WO93/09116, WO93/10073, WO93/14113, WO93/18023, WO93/19064, WO93/21155, WO93/21181, WO93/23380, WO93/24465, WO94/01402, WO94/02461, WO94/03429, WO94/03445, WO94/04494, WO94/04496, WO94/05625, WO94/07843, WO94/10165, WO94/10167, WO94/10168, WO94/10170, WO94/11368, WO94/13639, WO94/13663, WO94/14767, WO94/15903, WO94/19320, WO94/19323, WO94/20500, WO94/26735, WO94/26740, WO94/29309, WO95/02595, WO95/04040, WO95/04042, WO95/06645, WO95/07886, WO95/07908, WO95/08549, WO95/11880, WO95/14017, WO95/15311, WO95/16679, WO95/17382, WO95/18124, WO95/18129, WO95/19344, WO95/20575, WO95/121819, WO96/122525, WO95/123798, WO95/26338, WO95/28418, WO95/30674, WO95/30687, WO96/05193, WO96/05203, WO96/06094, WO96/07649, WO96/10562, WO96/16939, WO96/18643, WO96/20197, WO96/21661, WO96/29304, WO96/29317, WO96/129326, WO96/29328, WO96/31214, WO96/132385, WO96/37489, WO97/01553, WO97/01554, WO97/03066, WO97/08144, WO97/14671, WO97/17362, WO97/18206, WO97/19084, WO97/19942, WO97/21702 and the like, compound (I), compound (Ia) and salts thereof, and prodrugs thereof, to be described in detail in the following, novel NK-1 receptor antagonists to be found in the future and the like.

The compound (I) is explained in detail in the following.

**Regarding "Ring M, X and Y":**

In the above-mentioned formulae (I) and (Ia), Ring M is a heterocyclic ring having -N=C<, -CO-N< or -CS-N< as the partial structure:

— X $\overline{\text{----------}}$ Y< .

Preferably, Ring M has -CO-N< or -N=C< as the partial structure:

— X $\overline{\text{----------}}$ Y< .

**Regarding "$R^a$ and $R^b$":**

In the above-mentioned formula (I), $R^a$ and $R^b$ are bonded to each other to form Ring A, or these are the same or different and each represent a hydrogen atom or a substituent on the Ring M.

The substituents $R^a$ and $R^b$ on the Ring M include, for example, a halogen atom, an optionally substituted alkyl group, an optionally halogenated alkoxy group, an optionally halogenated alkylthio group, a cycloalkyl group, an aryl group, an acylamino group, an acyloxy group, a hydroxy group, a nitro group, a cyano group, an amino group, a mono- or di-alkylamino group, a cyclic amino group (e.g., a cyclic amino group optionally containing hetero atom(s) of oxygen atom, sulfur atom, etc., in addition to nitrogen atom), an alkylcarbonylamino group, an alkylsulfonylamino group, an alkoxycarbonyl group, a carboxyl group, an alkylcarbonyl group, a carbamoyl group, a mono- or di-alkylcarbamoyl group, an alkylsulfonyl group, an oxo group, etc.

The above-mentioned "halogen atom" includes, for example, fluorine, chlorine, bromine and iodine atoms. Preferably, the halogen atom includes, for example, fluorine, chlorine and bromine atoms.

The "optionally substituted alkyl group" includes, for example, $C_{1-6}$ alkyl groups (e.g., methyl, ethyl, propyl, isopropyl, butyl, isobutyl, sec-butyl and tert-butyl groups, etc.) optionally having from 1 to 5 substituents selected from a hydroxy group, a $C_{1-6}$ alkoxy group (e.g., methoxy, ethoxy, propoxy, butoxy, isobutoxy, sec-butoxy, tert-butoxy, etc.), a $C_{1-6}$ alkylthio group (e.g., methylthio, ethylthio, propylthio, butylthio, isobutylthio, sec-butylthio, tert-butylthio, etc.), an amino group, a $C_{1-7}$ acylamino group (e.g. formylamino, acetylamino, propionylamino, butyrylamino, benzoylamino, etc.), an N-alkylamino group, a carboxyl group, a nitro group, a mono- or di-$C_{1-6}$ alkylamino group (e.g., methylamino, ethylamino, propylamino, butylamino, dimethylamino and diethylamino groups, etc.), an optionally substituted N-substituted amino group substituted by one or. two homocyclic ring (e.g., mono- or di- $C_{3-8}$ cycloalkylamino groups, for example, cyclopropylamino, cyclobutylamino, cyclohexylamino; $C_{6-10}$ arylamino groups, for example, phenylamino, etc.), an optionally substituted heterocyclic groups [e.g., 5-membered to 9-membered cyclic amino groups which may have 1 to 3 hetero atoms selected from oxygen atom, sulfur atom and the like in addition to nitrogen atom (e.g., 5-membered or 6-membered non-aromatic cyclic amino groups, for example, piperidino, 4-methylpiperidino, morpholino, thiomorpholino, piperazinyl, 4-methylpiperazinyl, 4-ethylpiperazinyl, pyrrolidinyl, imidazolydinyl, pyrazolydinyl; 5-membered or 6-membered aromatic cyclic amino groups, for example, pyridyl, pyradyl, pyrimidinyl, pyridazinyl, pyrrolyl, imidazolyl, pyrazolyl, etc.), aromatic heterocyclic rings (e.g., thiophenyl, furanyl, thiazole, isothiazole, oxazole, isoxazole, etc.), non-aromatic heterocyclic rings (e.g., tetrahydropyridyl, dihydropyridyl, tetrahydropyradyl, tetrahydropyrimidinyl, tetrahydropyridazinyl, dihydropyranyl, dihydropyrrolyl, dyhydroimidazolyl, dihydropyrazolyl, dihydrothiophenyl, dihydrofuranyl, dihydrothiazolyl, dihydroisothiazolyl, dihydrooxazolyl, dihydroisooxazolyl, hexahydropyrimidinyl, hexahydropyridazinyl, tetrahydropyranyl, pyrazolydinyl, tetrahydrothiophenyl, tetrahydrofuranyl, tetrahydrothiazolyl, tetrahydroisothiazolyl, tetrahydrooxazolyl, tetrahydroisooxazolyl, etc.)], an alkylsulfonylamino groups (e.g. $C_{1-4}$ alkylsulfonylamino groups, for example, methylsulfonylamino, ethylsulfonylamino, etc.), a $C_{1-6}$ alkyl-carbonyloxy group (e.g., $C_{1-4}$ alkyl-carbonyloxy group, for example, acetoxy, ethylcarbonyloxy, propylcarbonyloxy and butylcarbonyloxy groups, etc.) and a halogen atom (e.g., fluorine, chlorine and bromine atoms, etc.), etc.

Preferably, the "optionally substituted alkyl group" includes $C_{1-6}$ alkyl groups optionally substituted by from 1 to 4 or so halogen atoms, especially optionally halogenated $C_{1-4}$ alkyl groups (e.g., $C_{1-4}$ alkyl groups and $C_{1-4}$ alkyl groups substituted by from 1 to 5 (particularly from 1 to 3) or so halogen atoms, etc., such as methyl, chloromethyl, fluoromethyl, difluoromethyl, trichloromethyl, trifluoromethyl, ethyl, 2-bromoethyl, 2,2,2-trichloroethyl, 2,2,2-trifluoroethyl, pentafluoroethyl, propyl, 3,3,3-trifluoropropyl, isopropyl, 1-(trifluoromethyl)ethyl, butyl, 4,4,4-trifluorobutyl, isobutyl, sec-butyl and tert-butyl groups, etc.),

$C_{1-6}$ alkoxy-$C_{1-6}$ alkyl groups (e.g. $C_{1-4}$ alkoxy-$C_{1-4}$ alkyl groups, for example, methoxymethyl, ethoxymethyl, isopropoxymethyl, butoxymethyl, methoxyethyl, ethoxyethyl, etc.),

$C_{1-6}$ alkyltho-$C_{1-6}$ alkyl groups (e.g. $C_{1-4}$ alkylthio-$C_{1-4}$ alkyl groups, for example, methylthiomethyl, ethylthiomethyl, butylthiomethyl, methylthioethyl, ethylthioethyl, etc.), amino-$C_{1-6}$ alkyl groups (preferably, amino-$C_{1-4}$ alkyl groups), for example, aminomethyl, 2-aminoethyl, 2-aminopropyl, 3-aminopropyl, 2-aminobutyl, 3-aminobutyl and 4-aminobutyl groups, etc.),

$C_{1-7}$ acylamino-$C_{1-6}$ alkyl groups (e.g. $C_{1-7}$ acylamino-$C_{1-4}$ alkyl groups, for example, formylaminomethyl, acetylaminomethyl, propionylaminomethyl, formylaminoethyl, acetylaminoethyl, propionylaminoethyl, butylylaminoethyl, benzoylaminomethyl, etc.),

mono- or di-$C_{1-6}$ alkylamino-$C_{1-6}$ alkyl groups (e.g. mono- or di-$C_{1-4}$ alkylamino-$C_{1-4}$ alkyl groups, for example, methylaminomethyl, ethylaminomethyl, butylaminomethyl, dimethylaminomethyl, diethylaminomethyl, 2-(N-methylamino) ethyl, 2-(N-ethylamino)ethyl, 2-(N-methylamino)propyl, 3-(N-methylamino)propyl, 3-(N-methylamino)butyl, 4-(N-methylamino)butyl, 2-(N-dimethylamino)ethyl, 2-(N-dimethylamino)ethyl groups, $C_{3-10}$ cycloalkylamino-$C_{1-6}$ alkyl groups (e. g. $C_{3-8}$ cycloalkylamino-$C_{1-4}$ alkyl groups, for example, cyclopropylaminomethyl, cyclobutylaminomethyl, cyclohexylaminomethyl, cyclopropylaminoethyl, cyclobutylaminoethyl, cyclohexylaminoethyl, etc.), $C_{6-10}$ arylamino-$C_{1-6}$ alkyl groups (e.g. $C_{6-10}$ arylamino-$C_{1-4}$ alkyl groups, for example, phenylaminomethyl group, etc.), 5-membered or 6-membered cyclic amino optionally having 1 to 3 hetero atoms selected from oxygen atom and sulfur atom in addition to nitrogen atom -$C_{1-6}$ alkyl groups (e.g. non-aromatic cyclic amino-$C_{1-4}$ alkyl groups, for example, piperidinomethyl, 4-methylpiperidinomethyl, morpholinomethyl, thiomorpholinomethyl, piperazinylmethyl, 4-methylpiperazinylmethyl, piperidinoethyl, morpholinoethyl, piperazinylethyl; 5-membered or 6-membered aromatic cyclic amino-$C_{1-4}$ alkyl groups, for example, pyridylmethyl, pyrimidinylmethyl, imidazolylmethyl, pyridylethyl, etc.), $C_{1-6}$ alkylsulfonylamino-$C_{1-6}$ alkyl groups (e.g. $C_{1-4}$ alkylsulfonylamino-$C_{1-6}$ alkyl groups, for example, methylsulfonylaminomethyl, ethylsulfonylaminomethyl, methylsulfonylaminoethyl, ethylsulfonylaminoethyl, etc.), $C_{1-6}$ alkyl-carbonyloxy-$C_{1-6}$ alkyl groups (e.g. $C_{1-4}$ alkyl-carbonyloxy-$C_{1-4}$ alkyl groups, for example, methylcarbonyloxymethyl, ethylcarbonyloxymethyl, butylcarbonyloxymethyl, methylcarbonyloxyethyl, ethylcarbonyloxyethyl, etc.), etc.

The "optionally halogenated alkoxy group" includes, for example, $C_{1-6}$ alkoxy groups or $C_{1-6}$ alkoxy groups substituted by from 1 to 5 or so halogen atoms, etc. Such alkoxy groups or halogenated alkoxy groups include, for example, methoxy, fluoromethoxy, difluoromethoxy, trifluoromethoxy, trichloromethoxy, ethoxy, 2,2,2-trifluoroethoxy, 2,2,2-trichloroethoxy, pentafluoroethoxy, propoxy, isopropoxy, butoxy, 4,4,4-trifluorobutoxy, isobutoxy, sec-butoxy, pentoxy and hexyloxy groups, etc. Preferably, the "optionally halogenated alkoxy group" includes $C_{1-4}$ alkoxy groups or $C_{1-4}$ alkoxy group substituted by from 1 to 3 or so halogen atoms, for example, methoxy, difluoromethoxy, trifluoromethoxy, ethoxy, 2,2,2-trifluoroethoxy, propoxy, isopropoxy, butoxy, 4,4,4-trifluorobutoxy, isobutoxy and sec-butoxy groups, etc.

The "optionally halogenated alkylthio group" includes, for example, $C_{1-6}$ alkylthio groups, and $C_{1-6}$ alkylthio groups having from 1 to 5 or so halogen atoms, etc. Such alkylthio groups and halogenated alkylthio groups include, for example, methylthio, difluoromethylthio, trifluoromethylthio, ethylthio, propylthio, isopropylthio, butylthio, 4,4,4-trifluorobutylthio, pentylthio and hexylthio groups, etc. Preferably, the "optionally halogenated alkylthio group" includes $C_{1-4}$ alkylthio groups, or $C_{1-4}$ alkylthio groups substituted by from 1 to 3 or so halogen atoms, for example, methylthio, difluoromethylthio, trifluoromethylthio, ethylthio, propylthio, isopropylthio, butylthio and 4,4',4-trifluorobutylthio groups, etc.

Furthermore, the "cycloalkyl group" includes $C_{3-10}$ cycloalkyl groups (e.g., cyclopropyl, cyclobutyl, cyclopentyl, cyclohexyl and cyclooctyl groups, etc.); the "aryl group" includes $C_{6-10}$ aryl groups (e.g., phenyl group, etc.); the "acylamino group" includes, for example, $C_{1-7}$ acylamino groups (e.g., formylamino, acetylamino, propionylamino, butyrylamino and benzoylamino groups, etc.), etc. The "acyloxy group" includes, for example, $C_{1-3}$ acyloxy groups (e.g., formyloxy, acetoxy and propionyloxy groups, etc.), etc. The "mono- or di-alkylamino group" includes, for example, mono- or di-$C_{1-6}$ alkylamino groups (e.g., mono- or di-$C_{1-4}$ alkylamino groups such as methylamino, ethylamino, propylamino, dimethylamino and diethylamino groups, etc.), etc. The "cyclic amino group" includes, for example, 5-membered to 9-membered cyclic amino groups optionally having from 1 to 3 hetero atoms, such as oxygen atom, sulfur atom, etc., in addition to nitrogen atom (e.g., pyrrolidino, piperidino, morpholino and thiomorpholino groups, etc.), etc. The "alkylcarbonylamino group" includes, for example, $C_{1-6}$ alkyl-carbonylamino groups (e.g., $C_{1-4}$ alkyl-carbonylamino groups such as acetylamino, propionylamino and butyrylamino groups, etc.); the "alkylsulfonylamino group" includes, for example, $C_{1-6}$ alkylsulfonylamino groups (e.g., $C_{1-4}$ alkylsulfonylamino groups such as methylsulfonylamino and ethylsulfonylamino groups, etc.); the "alkoxycarbonyl group" includes, for example, $C_{1-6}$ alkoxy-carbonyl groups (e.g., $C_{1-4}$ alkoxy-carbonyl groups such as methoxycarbonyl, ethoxycarbonyl, propoxycarbonyl and butoxycarbonyl groups, etc.); the "alkylcarbonyl group" includes, for example, $C_{1-6}$ alkyl-carbonyl groups (e.g., formyl, methylcarbonyl, ethylcarbonyl and propylcarbonyl groups, etc.); the "mono- or di-alkylcarbamoyl group" includes for example, mono- or di-$C_{1-6}$ alkylcarbamoyl groups (e.g., mono- or di-$C_{1-4}$ alkylcarbamoyl groups such as methylcarbamoyl, ethylcarbamoyl, dimethylcarbamoyl and diethylcarbamoyl groups, etc.); the "alkylsulfonyl group" includes, for example, $C_{1-6}$ alkylsulfonyl groups (e.g., methylsulfonyl, ethylsulfonyl and propylsulfonyl groups, etc.), etc.

**Regarding "Ring A and Ring B":**

In the above-mentioned formulae (I) and (Ia), Ring A and Ring B represent, independently, an optionally substituted homocyclic ring or heterocyclic ring, and at least one of these is an optionally substituted heterocyclic ring.

The "homocyclic ring or heterocyclic ring" includes, for example, (i) an aromatic heterocyclic ring or non-aromatic heterocyclic ring having one or two kinds of hetero atoms selected from nitrogen, sulfur and oxygen atoms, preferably from 1 to 3 such hetero atoms, in addition to carbon atoms, or (ii) a cyclic hydrocarbon (homocyclic ring) consisting of carbon atoms, etc.

The "aromatic heterocyclic ring" includes, for example, 5-membered or 6-membered aromatic heterocyclic rings having 1 to 3 hetero atoms selected from nitrogen, oxygen and sulfur atoms, in addition to carbon atoms (e.g., pyridine, pyrazine, pyrimidine, pyridazine, pyrrole, imidazole, pyrazole, triazole, thiophene, furan, thiazole, isothiazole, oxazole and isoxazole rings, etc.), etc. Preferably, the aromatic heterocyclic ring includes, for example, pyridine, pyrazine and thiophene rings, etc., as well as pyrrole and thiazole rings, etc. Especially preferred are (i) 6-membered, nitrogen-containing heterocyclic rings having one or two nitrogen atoms in addition to carbon atoms (e.g., pyridine and pyrazine rings, etc.) or (ii) 5-membered aromatic heterocyclic rings having one sulfur atom in addition to carbon atoms (e.g., thiophene ring, etc.), etc.

The "non-aromatic heterocyclic ring" includes, for example, 5-membered to 9-membered, non-aromatic heterocyclic rings, preferably 5-membered or 6-membered, non-aromatic heterocyclic rings, having from 1 to 3 hetero atoms selected from nitrogen, oxygen and sulfur atoms in addition to carbon atoms, etc.

For example, Ring A includes tetrahydropyridine, dihydropyridine, tetrahydropyrazine, tetrahydropyrimidine, tetrahydropyridazine, dihydropyran, dihydropyrrole, dihydroimidazole, dihydropyrazole, dihydrothiophene, dihydrofuran, dihydrothiazole, dihydroisothiazole, dihydroxazole and dihydroisoxazole rings, etc.; and Ring B includes, in addition to the above mentioned rings, piperidine, piperazine, hexahydropyrimidine, hexahydropyridazine, tetrahydropyran, morpholine, pyrrolidine, imidazolidine, pyrazolidine, tetrahydrothiophene, tetrahydrofuran, tetrahydrothiazole, tetrahydroisothiazole, tetrahydroxazole and tetrahydroisoxazole rings, etc. Preferably, Ring A includes, for example, 6-membered, non-aromatic heterocyclic rings having one or two nitrogen atoms in addition to carbon atoms (e.g., tetrahydropyridine, tetrahydropyrimidine and tetrahydropyridazine rings, etc.), etc., and is especially commonly used a tetrahydropyridine ring, etc. Preferably, Ring B includes, for example, 6-membered, non-aromatic heterocyclic rings having one or two nitrogen atoms in addition to carbon atoms (e.g., piperidine and piperazine rings, etc.), etc., and is especially commonly used a piperazine ring, etc.

The "cyclic hydrocarbon (homocyclic ring)" includes, for example, 3-membered to 10-membered (for example, 5-membered to 9-membered) cyclic hydrocarbon, preferably 5-membered or 6-membered cyclic hydrocarbon, etc. For example, Ring A includes benzene, $C_{3-10}$ cycloalkenes (e.g., cyclobutene, cyclopentene, cyclohexene, cycloheptene, cyclooctene, etc.), etc. The cycloalkenes are preferably $C_{5-6}$ cycloalkenes (e.g., cyclopentene, cyclohexene, etc.), etc.

Ring B includes, in addition to the above-mentioned rings, $C_{3-10}$ cycloalkanes (e.g., cyclobutane, cyclopentane, cyclohexane, cycloheptane, cyclooctane, etc.), etc. The cycloalkanes are preferably $C_{5-6}$ cycloalkanes (e.g., cyclohexane, cyclopentane, etc.), etc. Preferably, Ring A includes, for example, 6-membered homocyclic rings such as benzene and cyclohexene rings, etc. Especially preferred are a benzene ring, etc. Ring B preferably includes, for example, 6-membered homocyclic rings such as benzene and cyclohexane rings, etc. Especially preferred is a benzene ring.

At least one of Ring A and Ring B is an optionally substituted heterocyclic ring. Both of Ring A and Ring B may be optionally substituted heterocyclic rings. Preferably, one of Ring A and Ring B is 1) an optionally substituted aromatic ring and the other is 2) an optionally substituted heterocyclic ring (preferably, aromatic heterocyclic ring).

The above-mentioned 1) "aromatic ring" includes, for example, (i) the above-mentioned "aromatic heterocyclic rings", namely, optionally substituted, 5-membered or 6-membered, aromatic heterocyclic rings having one or two kind of hetero atoms selected from nitrogen, sulfur and oxygen atoms, preferably from 1 to 3 such hetero atoms, in addition to carbon atoms (e.g., pyridine, pyrazine, pyrimidine, pyridazine, pyrrole, imidazole, pyrazole, triazole, thiophene, furan, thiazole, isothiazole, oxazole and isoxazole rings, etc.), or (ii) optionally substituted benzene rings.

For the substituents for the above-mentioned 1) "aromatic ring", for example, referred to are the same substituents as those for Ring A and Ring B which are mentioned hereinunder. The "aromatic heterocyclic ring" of the above-mentioned 2) "optionally substituted aromatic heterocyclic ring" includes, for example, the same aromatic heterocyclic rings as those in the above-mentioned "5-membered or 6-membered, aromatic heterocyclic ring". For the substituents for the above-mentioned 2) "optionally substituted aromatic heterocyclic ring", for example, referred to are the same substituents as those for Ring A and Ring B which are mentioned hereinunder. The "5-membered or 6-membered, aromatic heterocyclic ring" preferably includes the same heterocyclic rings as those in the above-mentioned "aromatic heterocyclic ring".

More preferably, one of Ring A and Ring B is an optionally substituted aromatic heterocyclic ring (e.g., a 5-membered or 6-membered aromatic heterocyclic ring) and the other is an optionally substituted benzene ring.

The substituents for the optionally substituted "homocyclic ring or heterocyclic ring", "aromatic heterocyclic ring", "non-aromatic heterocyclic ring", "cyclic hydrocarbon", "aromatic ring" and "benzene ring" to be represented by Ring A and Ring B include, for example, a halogen atom, an optionally substituted alkyl group, an optionally halogenated alkoxy group, an optionally halogenated alkylthio group, an aryl group, an acylamino group, an acyloxy group, a hydroxy group, a nitro group, a cyano group, an amino group, a mono- or di-alkylamino group, a cyclic amino group (e.g., a cyclic amino group optionally having hetero atom selected from oxygen atom, sulfur atom, etc., in addition to nitrogen atom), an alkylcarbonylamino group, an alkylsulfonylamino group, an alkoxycarbonyl group, a carboxyl group, an alkylcarbonyl group, a carbamoyl group, a mono- or di-alkylcarbamoyl group, an alkylsulfonyl group, an oxo group, etc.

The "halogen atom", which Ring A and Ring B may have, includes, for example, fluorine, chlorine, bromine and iodine atoms. Preferably, the halogen atom includes, for example, fluorine, chlorine and bromine atoms (especially, fluorine and chlorine atoms, etc.).

The "optionally substituted alkyl group", which Ring A and Ring B may have, includes, for example, $C_{1-6}$ alkyl groups (e.g., methyl, ethyl, propyl, isopropyl, butyl, isobutyl, sec-butyl and tert-butyl groups, etc.) optionally having from 1 to 5 substituents selected from a hydroxy group, an amino group, a carboxyl group, a nitro group, a mono- or di-$C_{1-6}$ alkylamino group (e.g., methylamino, ethylamino, dimethylamino and diethylamino groups, etc.), a $C_{1-6}$ alkyl-carbonyloxy group (e.g., acetoxy and ethylcarbonyloxy groups, etc.) and a halogen atom (e.g., fluorine, chlorine and bromine atoms, etc.), etc. Especially preferred are optionally halogenated alkyl groups, for example, $C_{1-6}$ alkyl groups, and $C_{1-6}$ alkyl groups substituted by from 1 to 4 or so halogen atoms, etc. Such alkyl groups and halogenated alkyl groups include, for example, methyl, chloromethyl, fluoromethyl, difluoromethyl, trichloromethyl, trifluoromethyl, ethyl, 2-bromoethyl, 2,2,2-trichloroethyl, 2,2,2-trifluoroethyl, pentafluoroethyl, propyl, 3,3,3-trifluoropropyl, isopropyl, 1-(trifluoromethyl)ethyl, butyl, 4,4,4-trifluorobutyl, isobutyl, sec-butyl, tert-butyl, pentyl, isopentyl, neopentyl, 5,5,5-trifluoropentyl, 4-trifluoromethylbutyl, hexyl, 6,6,6-trifluorohexyl and 5-trifluoromethylpentyl groups, etc.

More preferably, the "optionally substituted alkyl group" includes optionally halogenated $C_{1-4}$ alkyl groups, for example, $C_{1-4}$ alkyl groups and $C_{1-4}$ alkyl groups substituted by from 1 to 3 or so halogen atoms, etc., such as methyl, chloromethyl, difluoromethyl, trichloromethyl, trifluoromethyl, ethyl, 2-bromoethyl, 2,2,2-trifluoroethyl, pentafluoroethyl, propyl, 3,3,3-trifluoropropyl, isopropyl, 2-trifluoromethylethyl, 1-(trifluoromethyl)ethyl, butyl, 4,4,4-trifluorobutyl, isobutyl, sec-butyl and tert-butyl groups, etc.

The "optionally halogenated alkoxy group", which Ring A and Ring B may have, includes, for example, $C_{1-6}$ alkoxy groups or $C_{1-6}$ alkoxy groups substituted by from 1 to 5 or so halogen atoms such as those mentioned hereinabove, etc. Such alkoxy groups or halogenated alkoxy groups include, for example, methoxy, difluoromethoxy, trifluoromethoxy, trichloromethoxy, ethoxy, 2,2,2-trifluoroethoxy, 2,2,2-trichloroethoxy, pentafluoroethoxy, propoxy, isopropoxy, butoxy, 4,4,4-trifluorobutoxy, isobutoxy, sec-butoxy, pentoxy and hexyloxy groups, etc. Preferably, the "optionally halogenated alkoxy group" includes $C_{1-4}$ alkoxy groups or $C_{1-4}$ alkoxy group substituted by from 1 to 3 or so halogen atoms, for example, methoxy, difluoromethoxy, trifluoromethoxy, ethoxy, 2,2,2-trifluoroethoxy, propoxy, isopropoxy, butoxy, 4,4,4-trifluorobutoxy, isobutoxy and sec-butoxy groups, etc.

The "optionally halogenated alkylthio group", which Ring A and Ring B may have, includes, for example, $C_{1-6}$ alkylthio groups, and $C_{1-6}$ alkylthio groups having from 1 to 5 or so halogen atoms such as those mentioned hereinabove, etc. Such alkylthio groups and halogenated alkylthio groups include, for example, methylthio, difluoromethylthio, trifluoromethylthio, ethylthio, propylthio, isopropylthio, butylthio, 4,4,4-trifluorobutylthio, pentylthio and hexylthio groups, etc. Preferably, the "optionally halogenated alkylthio group" includes $C_{1-4}$ alkylthio groups, or $C_{1-4}$ alkylthio groups substituted by from 1 to 3 or so halogen atoms, for example, methylthio, difluoromethylthio, trifluoromethylthio, ethylthio, propylthio, isopropylthio, butylthio and 4,4,4-trifluorobutylthio groups, etc.

The aryl group as the substituent includes $C_{6-10}$ aryl groups (e.g., phenyl group, etc.); the acylamino group includes, for example, $C_{1-7}$ acylamino groups (e.g., formylamino, acetylamino, propionylamino, butyrylamino and benzoylamino groups, etc.), etc. The acyloxy group includes, for example, $C_{1-3}$ acyloxy groups (e.g., formyloxy, acetoxy and propionyloxy groups, etc.), etc. The mono- or di-alkylamino group includes, for example, mono- or di-$C_{1-6}$ alkylamino groups (e.g., mono- or di-$C_{1-4}$ alkylamino groups such as methylamino, ethylamino, propylamino, dimethylamino and diethylamino groups, etc.), etc. The cyclic amino group includes, for example, 5-membered to 9-membered cyclic amino groups optionally having from 1 to 3 hetero atoms, such as oxygen atom, sulfur atom, etc., in addition to nitrogen atom (e.g., pyrrolidino, piperidino and morpholino groups, etc.), etc. The alkylcarbonylamino group includes, for example, $C_{1-6}$ alkyl-carbonylamino groups (e.g., $C_{1-4}$ alkyl-carbonylamino groups such as acetylamino, propionylamino and butyrylamino groups, etc.); the alkylsulfonylamino group includes, for example, $C_{1-6}$ alkylsulfonylamino groups (e.g., $C_{1-4}$ alkylsulfonylamino groups such as methylsulfonylamino and ethylsulfonylamino groups, etc.); the alkoxycarbonyl group includes, for example, $C_{1-6}$ alkoxy-carbonyl groups (e.g., $C_{1-4}$ alkoxy-carbonyl groups such as methoxycarbonyl, ethoxycarbonyl, propoxycarbonyl and butoxycarbonyl groups, etc.); the alkylcarbonyl group includes, for example, $C_{1-6}$ alkyl-carbonyl groups (e.g., formyl, methylcarbonyl, ethylcarbonyl and propylcarbonyl groups, etc.); the mono- or di-alkylcarbamoyl group includes, for example, mono- or di-$C_{1-6}$ alkylcarbamoyl groups (e.g., mono- or di-$C_{1-4}$ alkylcarbamoyl groups such as methylcarbamoyl, ethylcarbamoyl, dimethylcarbamoyl and diethylcarbamoyl groups, etc.); the. alkylsulfonyl group includes, for example, $C_{1-6}$ alkylsulfonyl groups (e.g., methylsulfonyl, ethylsulfonyl and propylsulfonyl groups, etc.), etc.

The terminology "optionally halogenated" as referred to herein means that the number of halogen atoms is from 1 to 5, preferably from 1 to 3 or so.

Preferred substituents which Ring A and Ring B may have include a halogen atom, an optionally halogenated $C_{1-4}$ alkyl group, an optionally halogenated $C_{1-4}$ alkoxy group, an optionally halogenated $C_{1-4}$ alkylthio group, a $C_{1-3}$ acyloxy group, a hydroxy group, an amino group, a mono- or di-$C_{1-4}$ alkylamino group, a carboxyl group, a $C_{1-4}$ alkoxycarbonyl group, an oxo group, etc.

More preferred substituents which Ring A and Ring B may have include a halogen atom, an optionally halogenated $C_{1-4}$ alkyl group, an optionally halogenated $C_{1-4}$ alkoxy group, a hydroxy group, an amino group, a mono- or di-$C_{1-4}$ alkylamino group, a $C_{1-3}$ acyloxy group, an oxo group, etc. Especially preferred are a halogen atom, an optionally halogenated $C_{1-4}$ alkyl group, an optionally halogenated $C_{1-4}$ alkoxy group, etc.

The substituents for Ring A and Ring B may be at any substitutable position on the ring. If the rings are substituted by two or more substituents, the substituents may be the same or different. The number of the substituents may be from 1 to 4 or so, preferably from 1 to 3 or so.

If the Ring A and/or the Ring B has(have) nitrogen atom(s), the ring may form a quaternary salt. For example, it may form a salt with halide ion(s) (e.g., $Cl^-$, $Br^-$, $I^-$, etc.) or other anion(s) such as sulfate ion, hydroxy ion, etc.

**Regarding "Ring A":**

Preferred homocyclic rings for Ring A are optionally substituted homocyclic rings composed of carbon atoms, for example, including those of a formula (A-1):

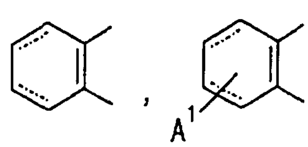

(A-1)

wherein ---------- indicates a single bond or a double bond and the same shall apply hereinunder; and $A^1$ represents a halogen atom (e.g., fluorine and chlorine atoms, etc.), an optionally halogenated $C_{1-4}$ alkyl group (e.g., methyl, isopropyl, trifluoromethyl, trichloromethyl, ethyl, 2,2,2-trifluoroethyl and pentafluoroethyl groups, etc.), or an optionally halogenated $C_{1-4}$ alkoxy group (e.g., methoxy, trifluoromethoxy, trichloromethoxy, ethoxy, 2,2,2-trifluoroethoxy and pentafluoroethoxy groups, etc.);
or those of a formula (A-2):

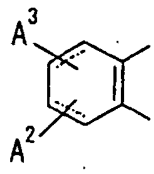

(A-2)

wherein $A^2$ and $A^3$ are the same or different and each represent a halogen atom (e.g., fluorine and chlorine atoms, etc.), an optionally halogenated $C_{1-4}$ alkyl group (e.g., methyl, isopropyl, trifluoromethyl, trichloromethyl, ethyl, 2,2,2-trifluoroethyl and pentafluoroethyl groups, etc.), or an optionally halogenated $C_{1-4}$ alkoxy group (e.g., methoxy, trifluoromethoxy, trichloromethoxy, ethoxy, 2,2,2-trifluroroethoxy and pentafluoroethoxy groups, etc.). More preferred homocyclic rings include, for example, homocyclic rings (especially benzene ring) of a formula (A-3):

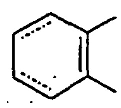 or 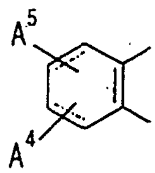

(A-3)

wherein $A^4$ and $A^5$ are the same or different and each represent a halogen atom (e.g., fluorine and chlorine atoms, etc.), or an optionally halogenated $C_{1-4}$ alkyl group (e.g., methyl, trifluoromethyl, trichloromethyl, ethyl, 2,2,2-trifluoroethyl, pentafluoroethyl and isopropyl groups, etc.).

Also, as homocyclic rings, preferred are optionally substituted benzene rings of a formula (A-4):

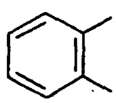 , 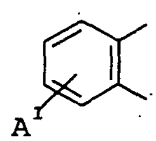 or 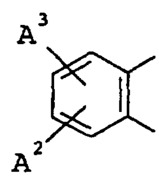

particularly

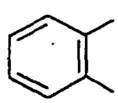 or 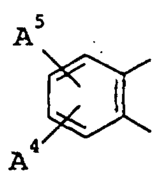

(A-4)

wherein the symbols are as defined above.

Of the homocyclic rings of the above-mentioned formulae, especially preferred are those as substituted by the following substituent(s):

(1) Homocyclic rings wherein $A^1$ is a halogen atom (e.g., fluorine and chlorine atoms, etc.), or an optionally halogenated $C_{1-4}$ alkyl group (e.g., methyl, trifluoromethyl, ethyl and isopropyl groups, etc.).
(2) Homocyclic rings wherein $A^2$ and $A^3$ are the same or different and each represent an optionally halogenated $C_{1-4}$ alkyl group (e.g., methyl, trifluoromethyl, ethyl and isopropyl groups, etc.), or an optionally halogenated $C_{1-4}$ alkoxy group (e.g., methoxy, trifluoromethoxy and ethoxy groups, etc.).
(3) Homocyclic rings wherein $A^4$ and $A^5$ are the same or different and each represent a $C_{1-4}$ alkyl group (e.g.,

methyl, ethyl and isopropyl groups, etc.).

(4) Homocyclic rings wherein $A^1$ is a halogen atom (e.g., fluorine and chlorine atoms, etc.).

(5) Homocyclic rings wherein $A^2$ and $A^3$ are the same or different and each represent a $C_{1-4}$ alkoxy group (e.g., methoxy and ethoxy groups, etc.).

Preferred aromatic heterocyclic or non-aromatic heterocyclic rings for Ring A are 5-membered or 6-membered, aromatic heterocyclic or non-aromatic heterocyclic rings including, for example, pyridine, pyrazine, thiophene, tetrahydropyridine, pyrrole and thiazole rings, etc. Concretely, for example, preferred are heterocyclic rings of a formula (A-5):

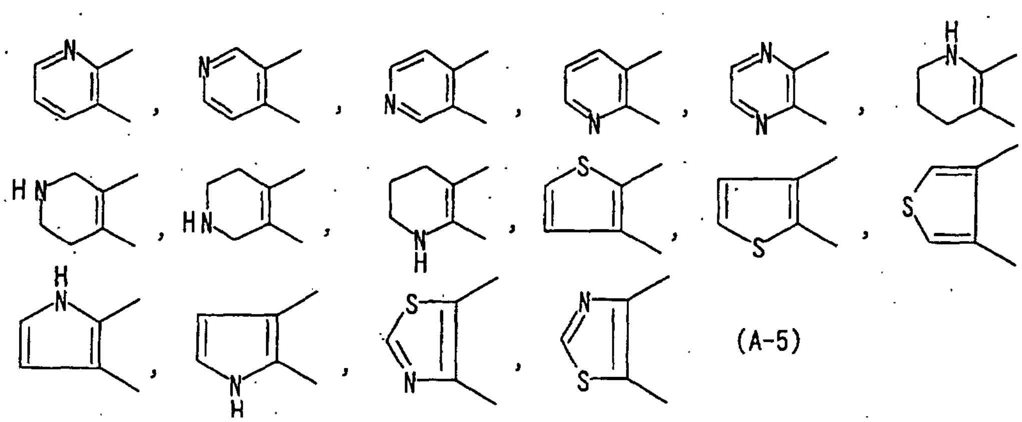

(A-5)

As preferred examples of optionally substituted aromatic or non-aromatic heterocyclic rings, mentioned are pyridine, pyrazine, thiophene, tetrahydropyridine, pyrrole and thiazole rings, etc. optionally having one or two substituents selected from an oxo group, an optionally substituted alkyl group (this has the same meaning as the substituent which Ring A and Ring B may have), a $C_{6-10}$ aryl group (e.g., phenyl group, etc.) and a halogen atom (e.g., fluorine, chlorine and bromine atoms, etc.). Concretely, for example, preferred are aromatic or non-aromatic heterocyclic rings of a formula (A-6):

(i) 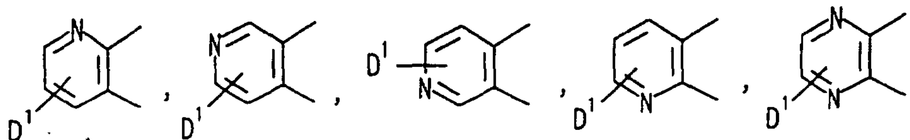 ;

(ii) 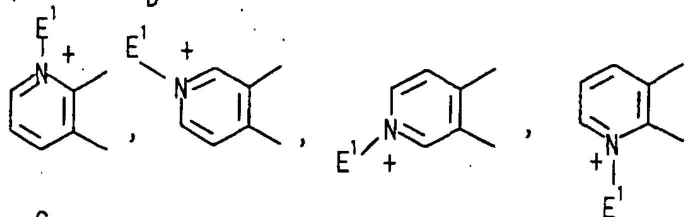 ;

(iii) 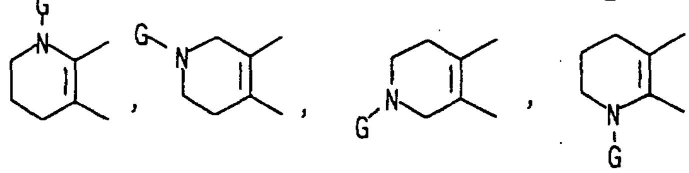 ;

(iv) 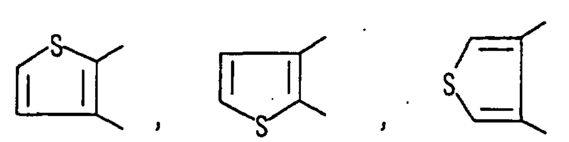 ;

(v) 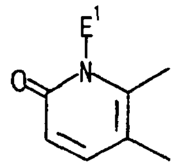 ;

(vi) 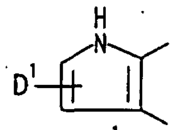 , 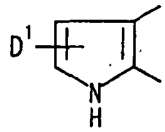 ;

(vii) 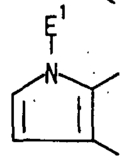 , 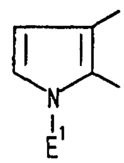

or

(viii) 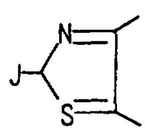 , 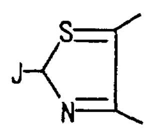 (A-6)

wherein $D^1$ represents a hydrogen atom, a halogen atom (e.g., fluorine, chlorine and bromine atoms, etc.); $E^1$ represents a $C_{1-4}$ alkyl group (e.g., methyl, ethyl, propyl and isopropyl groups, etc.); the compounds having the partial structure of (ii) form quaternary ammonium salts along with a halide ion (e.g., $Cl^-$, $Br^-$, $I^-$, etc.), a sulfate ion, a hydroxy ion or the like; G represents a hydrogen atom or a $C_{1-4}$ alkyl group (e.g., methyl, ethyl, propyl and isopropyl groups, etc.); J represents a hydrogen atom, a $C_{1-4}$ alkyl group (e.g., methyl, ethyl, propyl and isopropyl groups, etc.) or a $C_{6-10}$ aryl group (e.g., phenyl group, etc.).

Ring A is preferably a 5-membered or a 6-membered, nitrogen-containing heterocyclic ring, for example, (i) a 6-membered, aromatic, nitrogen-containing heterocyclic ring having one or two nitrogen atoms in addition to carbon atoms (e.g., pyridine and pyrazine rings, etc.), (ii) a 6-membered, non-aromatic heterocyclic ring having one or two nitrogen atoms in addition to carbon atoms (e.g., tetrahydropyridine, tetrahydropyrimidine and tetrahydropyridazine rings, etc.), or the like. Especially preferably, Ring A is an aromatic, nitrogen-containing heterocyclic ring, particularly, a pyridine ring or the like.

**Regarding "Ring B":**

Preferred homocyclic rings for Ring B are optionally substituted homocyclic rings consisting of carbon atoms, for example, including those of a formula (B-1):

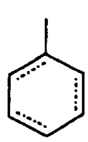 , 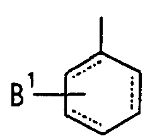 (B-1)

wherein $B^1$ represents a halogen atom, a $C_{1-4}$ alkyl group optionally substituted by hydroxy or optionally halogenated, an optionally halogenated $C_{1-4}$ alkoxy group, a $C_{1-6}$ alkyl-carbonyl group or a carboxyl group; those of a formula (B-2):

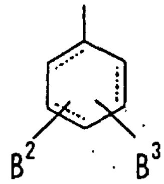

(B-2)

wherein $B^2$ and $B^3$ are the same or different and each represent a halogen atom, an optionally halogenated $C_{1-4}$ alkyl group or an optionally halogenated $C_{1-4}$ alkoxy group; and those of a formula (B-3):

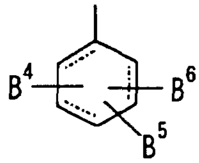

(B-3)

wherein $B^4$, $B^5$ and $B^6$ are the same or different and each represent a halogen atom, an optionally halogenated $C_{1-4}$ alkyl group or an optionally halogenated $C_{1-4}$ alkoxy group.

More preferred are homocyclic rings of a formula (B-4):

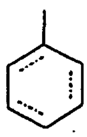

,

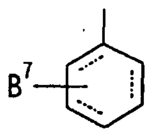

or

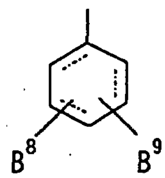

(B-4)

wherein $B^7$, $B^8$ and $B^9$ are the same or different and each represent a halogen atom, an optionally halogenated $C_{1-4}$ alkyl group or an optionally halogenated $C_{1-4}$ alkoxy group.

Even more preferred are homocyclic rings of a formula (B-5):

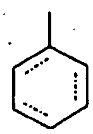

or

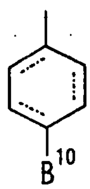

(B-5)

wherein $B^{10}$ represents, a halogen atom, a $C_{1-4}$ alkyl group optionally substituted by hydroxy or optionally halogenated, an optionally halogenated $C_{1-4}$ alkoxy group, a $C_{1-6}$ alkyl-carbonyl group or a carboxyl group.

In the above-mentioned formulae, the halogen atom for any of $B^1$ to $B^{10}$ includes, for example, fluorine, chlorine and bromine atoms, etc.; the optionally halogenated $C_{1-4}$ alkyl group includes, for example, methyl, trifluoromethyl, trichloromethyl, ethyl, 2,2,2-trifluoroethyl, 2,2,2-trichloroethyl, 1,1,2,2-tetrafluoroethyl, pentafluoroethyl, propyl, 2,2,3,3-tetrafluoropropyl and isopropyl groups, etc.; and the optionally halogenated $C_{1-4}$ alkoxy group includes, for example, methoxy, trifluoromethoxy, trichloromethoxy, ethoxy, 2,2,2-trifluoroethoxy, 2,2,2-trichloroethoxy, 1,1,2,2-tetrafluoroethoxy, pentafluoroethoxy, propoxy, 2,2,3,3-tetrafluoropropoxy and isopropoxy groups, etc.

In the above-mentioned formulae, the $C_{1-6}$ alkyl-carbonyl group includes, for example, formyl, acetyl.

Ring B is also preferably an optionally substituted benzene ring, which includes, for example, benzene rings of a formula (B-6):

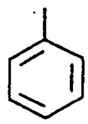, 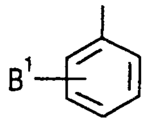, 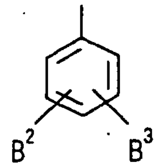 or 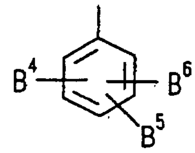 (B-6)

More preferred are benzene rings of a formula (B-7):

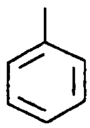, 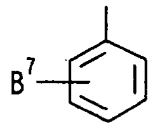 or 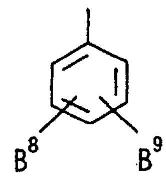 (B-7)

Especially preferred are benzene rings of a formula (B-8):

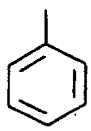 or 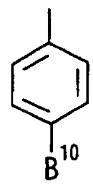 (B-8)

In these formulae, the symbols are as defined above.
Of the substituents in the above-mentioned formulae, for example, especially preferred are the following:

(1) $B^1$, $B^2$, $B^3$, $B^4$, $B^5$ and $B^6$ are the same or different and each represent a halogen atom (e.g., fluorine and chlorine atoms, etc.) or an optionally halogenated $C_{1-4}$ alkyl group (e.g., methyl, trifluoromethyl, ethyl and isopropyl groups, etc.).
(2) $B^1$, $B^2$, $B^3$, $B^4$, $B^5$ and $B^6$ are the same or different and each represent an optionally halogenated $C_{1-4}$ alkoxy group (e.g., methoxy, trifluoromethoxy and ethoxy groups, etc.).
(3) $B^7$, $B^8$ and $B^9$ represent halogen atoms (e.g., fluorine and chlorine atoms, etc.).
(4) $B^{10}$ represents a fluorine atom.
(5) $B^{10}$ represents a $C_{1-4}$ alkyl group (e.g., methyl group, etc.).
(6) $B^1$ or $B^{10}$ represents a $C_{1-6}$ alkyl group which may be substituted by hydroxy (e.g., hydroxymethyl, etc.), a $C_{1-6}$ alkyl-carbonyl group (e.g., formyl, acetyl, etc.), a carboxyl group.

More preferred optionally substituted benzene rings are phenyl groups and substituted phenyl group of a formula (B-9):

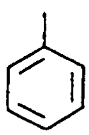, 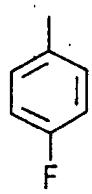 or 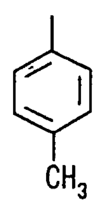 (B-9)

As preferred examples of aromatic heterocyclic rings or non-aromatic heterocyclic rings for Ring B, mentioned are 5-membered or 6-membered aromatic heterocyclic rings or non-aromatic heterocyclic rings such as pyridine, thiophene and piperidine rings, etc. These rings may optionally be substituted by substituents such as those mentioned hereinabove as preferred substituents for Ring A.
Where Ring B is an aromatic heterocyclic ring or a non-aromatic heterocyclic ring, it especially preferably

includes, for example, heterocyclic rings of a formula (B-10):

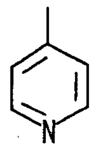 , 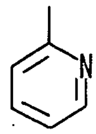 or 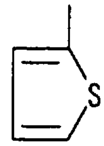 (B-10)

where one or both of Ring A and Ring B is/are heterocyclic ring(s), the ring(s) is/are also preferably unsubstituted one(s).

**Combination of Ring A and Ring B**

Preferred combination of Ring A and Ring B (1) is as follows:

(1) One of Ring A and Ring B is a 5-membered or 6-membered heterocyclic ring having one or two hetero atoms selected from nitrogen and sulfur atoms in addition to carbon atoms (e.g., pyridine, pyrazine, thiophene, tetrahydropyridine, piperidine and piperazine rings, etc.) which may be optionally substituted by $C_{1-4}$ alkyl group(s) (e.g., methyl, ethyl and isopropyl groups, etc.).

The other of Ring A and Ring B is a benzene ring optionally substituted by from 1 to 3 substituents selected from a halogen atom (e.g., fluorine, chlorine and bromine atoms, etc.), an optionally halogenated $C_{1-4}$ alkyl group (e.g., methyl, trifluoromethyl, trichloromethyl, ethyl, 2,2,2-trifluoroethyl, pentafluoroethyl, 2,2,2-trichloroethyl, propyl and isopropyl groups, etc.) and an optionally halogenated $C_{1-4}$ alkoxy group (e.g., methoxy, trifluoromethoxy, trichloromethoxy, ethoxy, 2,2,2-trifluoroethoxy, pentafluoroethoxy, 2,2,2-trichloroethoxy, propoxy and isopropoxy groups, etc.).
More preferred combination of Ring A and Ring B (2) is as follows:

(2) One of Ring A and Ring B is a 5-membered or 6-membered aromatic heterocyclic ring having one or two hetero atoms selected from nitrogen and sulfur atoms in addition to carbon atoms (e.g., pyridine, pyrazine and thiophene rings, etc.).

The other of Ring A and Ring B is a benzene ring optionally substituted by from 1 to 3 substituents selected from a halogen atom (e.g., fluorine, chlorine and bromine atoms, etc.), an optionally halogenated $C_{1-4}$ alkyl group (e.g. methyl, trifluoromethyl, trichloromethyl, ethyl, 2,2,2-trifluoroethyl, pentafluoroethyl, 2,2,2-trichloroethyl, propyl and isopropyl groups, etc.) and an optionally halogenated $C_{1-4}$ alkoxy group (e.g., methoxy, trifluoromethoxy trichloromethoxy, ethoxy, 2,2,2-trifluoroethoxy, pentafluoroethoxy, 2,2,2-trichloroethoxy, propoxy and isopropoxy groups, etc.).
Especially preferably, Ring A is an optionally substituted aromatic heterocyclic ring such as mentioned above (e.g., 5-membered or 6-membered aromatic heterocyclic ring, especially pyridine ring, etc.), while Ring B is an optionally substituted benzene ring.

**Regarding "Ring C":**

In the above-mentioned formulae, Ring C represents an optionally substituted homocyclic ring or an optionally substituted heterocyclic ring. The homocyclic ring or the heterocyclic ring may have from 1 to 5 or so, preferably from 1 to 3 or so substituents, which may be the same or different The substituents may be positioned at any position of the homocyclic ring or heterocyclic ring.
The homocyclic ring includes "cyclic hydrocarbon (homocyclic ring)" such as those as referred to hereinabove for "Ring A and Ring B", for example, from 3-membered to 10-membered cyclic hydrocarbon such as benzene, $C_{3-10}$ cycloalkenes (e.g., cyclobutene, cyclopentene, cyclohexene, cycloheptene, cyclooctene, etc.), $C_{3-10}$ cycloalkanes (e.g., cyclobutane, cyclopentane, cyclohexane, cycloheptane, cyclooctane, etc.), etc., preferably 5-membered or 6-membered cyclic hydrocarbon. Of these, preferred are 6-membered homocyclic rings, such as benzene, cyclohexene and cyclohexane rings, etc. Especially preferred is benzene ring.
The substituents for the homocyclic rings such as the above-mentioned benzene ring include, for example, a halogen atom (e.g., fluorine, chlorine, bromine and iodine atoms), an optionally halogenated $C_{1-10}$ alkyl group (e.g., methyl, chloromethyl, difluoromethyl trichloromethyl, trifluoromethyl, ethyl, 2-bromoethyl, 2,2,2-trifluoroethyl, perfluoroethyl, propyl, isopropyl, 3,3,3-trifluoropropyl, butyl, isobutyl, tert-butyl, perfluorobutyl, pentyl, hexyl, octyl and decyl groups, etc.), an amino-substituted $C_{1-4}$ alkyl group (e.g., aminomethyl and 2-aminoethyl groups, etc.), a mono- or di-$C_{1-4}$ alkylamino-substituted $C_{1-4}$ alkyl group (e.g., methylaminomethyl, dimethylaminomethyl, 2-methylaminoethyl

and 2-dimethylaminoethyl groups, etc.), a carboxyl-substituted $C_{1-4}$ alkyl group (e.g., carboxymethyl and carboxyethyl groups, etc.), a $C_{1-4}$ alkoxy-carbonyl-substituted $C_{1-4}$ alkyl group (e.g., methoxycarbonylethyl and ethoxycarbonylethyl groups, etc.), a hydroxy-substituted $C_{1-4}$ alkyl group (e.g., hydroxymethyl and hydroxyethyl groups, etc.), a $C_{1-4}$ alkoxy-substituted $C_{1-4}$ alkyl group (e.g., methoxymethyl, methoxyethyl and ethoxyethyl groups, etc.), a $C_{3-10}$ cycloalkyl group (e.g., cyclopropyl, cyclobutyl, cyclopentyl, cyclohexyl, cycloheptyl and cyclooctyl groups, etc.), a nitro group, a cyano group, a hydroxy group, an optionally halogenated $C_{1-10}$ alkoxy group (e.g., methoxy, difluoromethoxy, trichloromethoxy, trifluoromethoxy, ethoxy, 2,2,2-trifluoroethoxy, perfluoroethoxy, propoxy, isopropoxy, butoxy, isobutoxy, tert-butoxy, perfluorobutoxy, pentyloxy, hexyloxy, octyloxy and decyloxy groups, etc.), an optionally halogenated $C_{1-4}$ alkylthio group (e.g., methylthio, difluoromethylthio, trifluoromethylthio, ethylthio, propylthio, isopropylthio and butylthio groups, etc.), an amino group, a mono- or di-$C_{1-4}$ alkylamino group (e.g., methylamino, ethylamino, propylamino, dimethylamino and diethylamino groups, etc.), a cyclic amino group (e.g., a 5-membered to 9-membered cyclic amino group optionally having from 1 to 3 hetero atoms such as oxygen and sulfur atoms, etc., in addition to nitrogen atoms, concretely for example, pyrrolidino, piperidino and morpholino groups, etc.), a $C_{1-4}$ alkyl-carbonylamino group (e.g., acetylamino, propionylamino and butyrylamino groups, etc.), an aminocarbonyloxy group, $C_{1-3}$ acyloxy group (e.g., formyloxy, acetoxy and propionyloxy groups, etc.), a mono- or di-$C_{1-4}$ alkylaminocarbonyloxy group (e.g., methylaminocarbonyloxy, ethylaminocarbonyloxy, dimethylaminocarbonyloxy and diethylaminocarbonyloxy groups, etc.), a $C_{1-4}$ alkylsulfonylamino group (e.g., methylsulfonylamino, ethylsulfonylamino and propylsulfonylamino groups, etc.), a $C_{1-4}$ alkoxy-carbonyl group (e.g., methoxycarbonyl, ethoxycarbonyl, propoxycarbonyl, butoxycarbonyl and isobutoxycarbonyl groups, etc.), an aralkyloxycarbonyl group (e.g., $C_{7-19}$ aralkyloxycarbonyl group such as phenyl-$C_{1-4}$ alkyloxy-carbonyl (e.g., benzyloxycarbonyl etc.), etc.), a carboxyl group, a $C_{1-6}$ alkyl-carbonyl group (e.g., methylcarbonyl, ethylcarbonyl and butylcarbonyl groups, etc.), a $C_{3-6}$ cycloalkyl-carbonyl group (e.g., cyclohexylcarbonyl group, etc.), a carbamoyl group, a mono- or di-$C_{1-4}$ alkylcarbamoyl group (e.g., methylcarbamoyl, ethylcarbamoyl, propylcarbamoyl, butylcarbamoyl, diethylcarbamoyl and dibutylcarbamoyl groups, etc.), a $C_{1-6}$ alkylsulfonyl group (e.g., methylsulfonyl, ethylsulfonyl and propylsulfonyl groups, etc.), etc.

The heterocyclic Ring C may optionally be substituted, for example, by one 5-membered or 6-membered, aromatic monocyclic heterocyclic group (e.g., furyl, thienyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl and triazinyl groups, etc.), etc., and the aromatic monocyclic heterocyclic group may optionally be substituted by from 1 to 3 or so optionally halogenated $C_{1-4}$ alkyl groups (e.g., methyl, chloromethyl, difluoromethyl, trichloromethyl, trifluoromethyl, ethyl and isopropyl groups, etc.), etc.

As preferred substituents for the homocyclic Ring C (e.g., benzene ring, etc.), for example, mentioned are a halogen atom (e.g., fluorine, chlorine and bromine atoms, etc.), an optionally halogenated $C_{1-6}$ alkyl group (e.g., methyl, chloromethyl, difluoromethyl, trichloromethyl, trifluoromethyl, ethyl, 2-bromoethyl, 2,2,2-trifluoroethyl, perfluoroethyl, propyl, isopropyl, 3,3,3-trifluoropropyl, butyl, sec-butyl, tert-butyl and perfluorobutyl groups, etc.), a nitro group, a hydroxy group, an optionally halogenated $C_{1-6}$ alkoxy group (e.g., methoxy, difluoromethoxy, trifluoromethoxy, ethoxy, 2,2,2-trifluoroethoxy, perfluoroethoxy, propoxy, isopropoxy, 3,3,3-trifluoropropoxy and butoxy groups, etc.), an amino group, a mono- or di-$C_{1-4}$ alkylamino-substituted $C_{1-4}$ alkyl group (e.g., methylaminomethyl, dimethylaminomethyl, 2-methylaminoethyl and 2-dimethylaminoethyl groups, etc.), a mono- or di-$C_{1-4}$ alkylamino group (e.g., methylamino, ethylamino, dimethylamino and diethylamino groups; etc.), $C_{1-3}$ acyloxy group (e.g., acetoxy group, etc.), a $C_{1-4}$ alkoxy-carbonyl group (e.g., methoxycarbonyl and ethoxycarbonyl groups, etc.), a carboxyl group, a carbamoyl group, etc.

More preferred are a halogen atom (e.g., fluorine, chlorine and bromine atoms, etc.), an optionally halogenated $C_{1-4}$ alkyl group (e.g., methyl, chloromethyl, difluoromethyl, trichloromethyl, trifluoromethyl, ethyl, 2-bromoethyl, 2,2,2-trichloroethyl, 2,2,2-trifluoroethyl, perfluoroethyl, propyl, isopropyl and tert-butyl groups, etc.), an optionally halogenated $C_{1-4}$ alkoxy group (e.g., methoxy, trifluoromethoxy, ethoxy, 2,2,2-trichloroethoxy, 2,2,2-trifluoroethoxy, perfluoroethoxy and propoxy groups, etc.), a di-$C_{1-4}$ alkylamino group (e.g., dimethylamino and diethylamino groups, etc.), a $C_{1-3}$ acyloxy group (e.g., acetoxy group, etc.), a hydroxy group, etc. Preferably, the number of the substituents is, for example, from 1 to 3 or so.

Especially, preferred are a halogen atom (e.g., fluorine, chlorine and bromine atoms, etc.), an optionally halogenated $C_{1-4}$ alkyl group (e.g., methyl, chloromethyl, difluoromethyl, trichloromethyl, trifluoromethyl, ethyl, 2-bromoethyl, 2,2,2-trichloroethyl, 2,2,2-trifluoroethyl, perfluoroethyl, propyl, isopropyl and tert-butyl groups, etc.); an optionally halogenated $C_{1-4}$ alkoxy group (e.g., methoxy, trifluoromethoxy, ethoxy, 2,2,2-trichloroethoxy, 2,2,2-trifluoroethoxy, perfluoroethoxy and propoxy groups, etc.)

The "heterocyclic ring" of the "optionally substituted heterocyclic ring" includes, for example, from 5-membered to 10-membered heterocyclic rings having 1 to 4 hetero atoms of the same type or two different types, such as nitrogen, oxygen, sulfur atoms, etc., in addition to carbon atoms, etc. Concretely, the heterocyclic ring includes, for example;

(1) 5-membered or 6-membered, aromatic monocyclic heterocyclic rings, such as furyl, thienyl, pyrrolyl, oxazolyl,

isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl, etc.;

(2) 9-membered or 10-membered, aromatic, condensed heterocyclic rings, such as benzofuranyl, isobenzofuranyl, benzo[b]thienyl, indolyl, isoindolyl, 1H-indazolyl, benzimidazolyl, benzoxazolyl, 1,2-benzoisoxazolyl, benzothiazolyl, 1,2-benzoisothiazolyl, 1H-benzotriazolyl, quinolyl, isoquinolyl, cinnolinyl., quinazolinyl, quinoxalinyl, phthalazinyl, naphthyridinyl, purinyl, pteridinyl, carbazolyl, α-carbolinyl, β-carbolinyl, γ-carbolinyl, acridinyl, phenoxazinyl, phenothiazinyl, phenazinyl, phenoxathinyl, thianthrenyl, phenanthridinyl, phenanthrolinyl, indolizinyl, pyrrolo[1,2-b]pyridazinyl, pyrazolo[1,5-a]pyridyl, imidazo[1,2-b]pyridazinyl, imidazo[1,2-a]pyrimidinyl, 1,2,4-triazolo[4,3-a]pyridyl, 1,2,4-triazolo[4,3-b]pyridazinyl, etc.;

(3) 5-membered to 10-membered, non-aromatic heterocyclic rings, such as oxiranyl, azetidinyl, oxetanyl, thietanyl, pyrrolidinyl, tetrahydrofuryl, piperidyl, tetrahydropyranyl, morpholinyl, thiomorpholinyl, pyrazinyl, etc.

Of the above-mentioned heterocyclic rings (1) to (3), for example, 5-membered or 6-membered heterocyclic rings having from 1 to 3 hetero atoms, such as nitrogen, oxygen and sulfur atoms, etc., in addition to carbon atoms, are widely utilized. Such heterocyclic rings include, for example, furyl, thienyl, pyrrolyl, oxazolyl, isoxazolyl, imidazolyl, pyrazolyl, pyridyl, pyridazinyl, quinolyl, isoquinolyl, thiazolyl, thiadiazolyl, thiophenyl, etc.

As the substituents for the optionally substituted heterocyclic rings, mentioned are substituents such as those as referred to above-mentioned "optionally substituted homocyclic rings".

More preferably, Ring C includes optionally substituted benzene rings (especially, substituted benzene rings by substituent), for example, benzene rings optionally substituted by 1 to 3 substituents selected from a halogen atom, an optionally halogenated $C_{1-4}$ alkyl group, an optionally halogenated $C_{1-4}$ alkoxy group, a di-$C_{1-4}$ alkylamino group, a $C_{1-3}$ acyloxy group and a hydroxy group (especially, benzene rings substituted by such substituent(s)). Concretely, the preferred Ring C includes, for example, optionally substituted benzene rings of a formula (C-1):

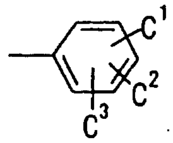

(C-1)

wherein $C^1$, $C^2$ and $C^3$ are the same or different and each represent a hydrogen atom, a halogen atom, an optionally halogenated $C_{1-4}$ alkyl group, an optionally halogenated $C_{1-4}$ alkoxy group, a mono- or di-$C_{1-4}$ alkylamino group, a $C_{1-3}$ acyloxy group or a hydroxy group; and

optionally substituted benzene rings of a formula (C-2):

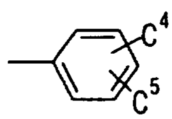

(C-2)

wherein $C^4$ and $C^5$ are the same or different and each represent a hydrogen atom, a halogen atom, an optionally halogenated $C_{1-4}$ alkyl group or an optionally halogenated $C_{1-4}$ alkoxy group.

The halogen atom, the optionally halogenated $C_{1-4}$ alkyl group, the optionally halogenated $C_{1-4}$ alkoxy group and the mono- or di-$C_{1-4}$ alkylamino group to be represented by any of $C^1$, $C^2$, $C^3$, $C^4$ and $C^5$ may be the same as the above-mentioned halogen atom, optionally halogenated $C_{1-4}$ alkyl group, optionally halogenated $C_{1-4}$ alkoxy group and mono- or di-$C_{1-4}$ alkylamino group, respectively.

Even more preferably, Ring C includes, for example, benzene rings of the above-mentioned formulae (C-1) and (C-2) where $C^1$ to $C^5$ are substituents mentioned below:

(1) $C^1$, $C^2$ and $C^3$ are the same or different and each represent a halogen atom, an optionally halogenated $C_{1-4}$ alkyl group or an optionally halogenated $C_{1-4}$ alkoxy group;

(2) $C^1$, $C^2$ and $C^3$ are the same or different and each represent a halogen atom or an optionally halogenated $C_{1-4}$ alkyl group;

(3) $C^1$, $C^2$ and $C^3$ are the same or different and each represent a halogen atom;

(4) $C^1$, $C^2$ and $C^3$ are the same or different and each represent an optionally halogenated $C_{1-4}$ alkyl group;

(5) $C^1$, $C^2$ and $C^3$ are the same or different and each represent an optionally halogenated $C_{1-4}$ alkoxy group;
(6) $C^4$ and $C^5$ are the same or different and each represent a halogen atom;
(7) $C^4$ and $C^5$ are the same or different and each represent an optionally halogenated $C_{1-4}$ alkyl group; or
(8) $C^4$ and $C^5$ are the same or different and each represent an optionally halogenated $C_{1-4}$ alkoxy group.

As examples of the "optionally halogenated $C_{1-4}$ alkyl group", the "optionally halogenated $C_{1-4}$ alkoxy group" and the "halogen atom" in the above-mentioned embodiments (1) to (8), referred to are the same groups or atoms as those mentioned hereinabove.
Furthermore preferably, Ring C includes, for example, benzene rings of the above-mentioned formula (C-2) wherein $C^4$ and $C^5$ are substituents mentioned below:

(a) one of $C^4$ and $C^5$ is a hydrogen atom and the other is a methoxy group;
(b) $C^4$ and $C^5$ are both chlorine atoms;
(c) one of $C^4$ and $C^5$ is a methoxy group and the other is an isopropyl group;
(d) one of $C^4$ and $C^5$ is a methoxy group and the other is a 1-methoxy-1-methylethyl group; or
(e) $C^4$ and $C^5$ are both trifluoromethyl groups.

**Regarding "Ring Z":**

In the above-mentioned formulae, Ring Z represents an optionally substituted nitrogen containing heterocyclic ring. Various substituents are referred to as substituents for Ring Z, which include, for example, an alkyl group (e.g., a linear or branched alkyl group having from 1 to 6 carbon atoms, preferably a linear or branched alkyl group having from 1 to 4 carbon atoms, such as methyl, ethyl, propyl, isopropyl, butyl, isobutyl, sec-butyl and tert-butyl groups, etc.), an alkenyl group (e.g., a $C_{2-6}$ alkenyl group, preferably a $C_{2-4}$ alkenyl group, such as ethenyl, propenyl, isopropenyl, butenyl, isobutenyl sec-butenyl groups, etc.), an alkynyl group (e.g., a $C_{2-6}$ alkynyl group, preferably a $C_{2-4}$ alkynyl group, such as ethynyl, propynyl, isopropynyl, butynyl, isobutynyl and sec-butynyl groups, etc.), a cycloalkyl group (e.g., a $C_{3-8}$ cycloalkyl group, preferably a $C_{3-8}$ cycloalkyl group, such as cyclopropyl, cyclobutyl, cyclopentyl and cyclohexyl groups, etc.), a cycloalkyl-alkyl group (e.g., a $C_{3-8}$ cycloalkyl-$C_{1-4}$ alkyl group, such as cyclopropylmethyl, cyclopropylethyl and cyclohexylmethyl groups, etc.), an aryl group (e.g., a $C_{6-14}$ aryl group, preferably a $C_{6-10}$ aryl group, such as phenyl, 1-naphthyl, 2-naphthyl, anthryl and phenanthryl groups, etc., especially, phenyl group), a nitro group, a cyano group, a hydroxy group, a $C_{1-4}$ alkoxy group (e.g., methoxy, ethoxy, propoxy, isopropoxy and butoxy groups, etc.), a $C_{1-4}$ alkylthio group (e.g., methylthio, ethylthio and propylthio groups, etc.), an amino group, a mono- or di-$C_{1-4}$ alkylamino group (e.g., methylamino, ethylamino, propylamino, dimethylamino and diethylamino groups, etc.), a cyclic amino group (e.g., a 5-membered to 9-membered cyclic amino group optionally having from 1 to 3 hetero atoms, such as oxygen and sulfur atoms, etc., in addition to nitrogen atom, concretely, for example, pyrrolidino, piperidino, morpholino and thiomorpholino groups, etc.), a $C_{1-4}$ alkyl-carbonylamino group (e.g., acetylamino, propionylamino and butyrylamino groups, etc.), a $C_{1-4}$ alkylsulfonylamino group (e.g., methylsulfonylamino and ethylsulfonylamino groups, etc.), a $C_{1-4}$ alkoxy-carbonyl group (e.g., methoxycarbonyl, ethoxycarbonyl and propoxycarbonyl groups, etc.), a carboxyl group, a $C_{1-6}$ alkyl-carbonyl group (e.g., methylcarbonyl, ethylcarbonyl and propylcarbonyl groups, etc.), a carbamoyl group, a mono- or di-$C_{1-4}$ alkylcarbamoyl group (e.g., methylcarbamoyl and ethylcarbamoyl groups, etc.), a $C_{1-6}$ alkylsulfonyl group (e.g., methylsulfonyl, ethylsulfonyl and propylsulfonyl groups, etc.), an oxo group, a thioxo group, etc. The number of the substituents is, for example, from 1 to 5 or so, preferably 1, 2 or so, depending on the size of Ring Z.
Ring Z may be a heterocyclic ring optionally having at least one hetero atom selected from nitrogen, oxygen and sulfur atoms, in addition to Y and the nitrogen atom N, and is preferably an optionally oxoated ring.
Ring Z includes rings of a formula (Z-1):

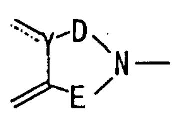 (Z-1)

wherein D and E represent groups from which Ring Z as mentioned above is formed together with the nitrogen atom adjacent to E.
At least one of, D and E which form Ring Z represent, independently, an optionally oxoated alkylene group, oxyalkylene group, or iminoalkylene group. preferable D and E are often optionally oxoated alkylene group and oxyalkylene group, respectively. The optionally oxoated alkylene groups, oxyalkylene group and iminoalkylene group,

which are represented by D and E preferably have carbon atoms from which Ring Z is formed to be a 5-membered to 12-membered ring, preferably a 5-membered to 9-membered ring. The numbers of the carbon atoms that constitute the alkylene groups of D and E may be the same or different.

Preferably, D includes, for example, optionally oxoated $C_{1-7}$ alkylene group, especially optionally oxoated $C_{1-5}$ alkylene group, $C_{1-7}$ oxyalkylene groups, especially $C_{1-5}$ oxyalkylene groups, $C_{1-7}$ iminoalkylene groups, especially $C_{1-5}$ imminoalkylene groups. More preferably, D includes an alkylene group of a formula $-(CH_2)_m-$ (wherein m is from 1 to 7), an oxyalkylene group of a formula $-O-(CH_2)_p-$ (wherein p is from 1 to 7), iminoalkylene group of a formula $-NH-(CH_2)_q-$ (wherein q is from 1 to 7). In these formulae, m and p are preferably from 1 to 5, more preferably from 2 to 5.

Preferably, E includes, for example, optionally oxoated $C_{1-3}$ alkylene group, more preferably an optionally oxoated alkylene group having one or two carbon atoms, even more preferably an optionally oxoated methylene group.

The number of the oxo groups that are substitutable in Ring Z is not specifically limited but may be selected from 1 to 3 or so depending on the size of Ring Z. Where Ring Z is a 5-membered to 10-membered ring, the number of the substitutable oxo groups is 1, 2 or so. Oxo group(s) may be substituted at at least either one of D and/or E. Preferably, oxo group(s) is/are substituted at E in Ring Z.

Preferably, in Ring Z, D is an alkylene group or oxyalkylene group having from 1 to 5 carbon atoms, more preferably from 2 to 5 carbon atoms, while E is an alkylene group having an oxo group having 1 or 2 carbon atoms, especially >C=O. Especially preferably, Ring Z includes, for example, from 5-membered to 9-membered rings of a formula (Z-2):

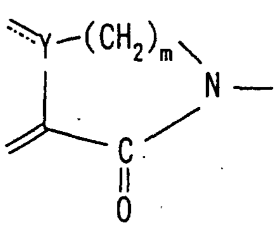

or

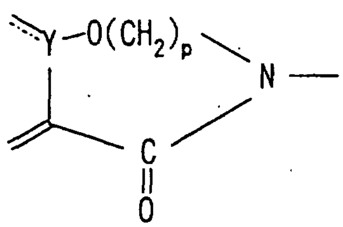

(Z-2)

wherein each m and p represents an integer of from 1 to 5.

**Regarding "n":**

In the above-mentioned formulae, n represents an integer of from 1 to 6, preferably an integer of from 1 to 3, especially preferably 1 or 2. More preferably, n is 1.

**Regarding compounds (I) and (Ia):**

In compounds represented by the above-mentioned general formulae (I) and (Ia), the combination of "Ring M",

"— X $\overline{\cdots\cdots}$ Y <" ,

"$R^a$", "$R^b$", "Ring A", "Ring B", "Ring C", "Ring Z" and "n" is not specifically limited. These may be combined suitably to construct the compounds (I) and (Ia). Preferred compounds (I) and (Ia) are constructed by combining the above-mentioned preferred embodiments of "Ring M",

" — X $\overline{\cdots\cdots}$ Y <" ,

"$R^a$", "$R^b$", "Ring A", "Ring B", "Ring C", "Ring Z" and "n".

Of compounds of the above-mentioned general formula (I), especially those of the above-mentioned general formula (Ia), preferred are (1) the following compounds or pharmaceutically-acceptable salts thereof.

Compounds wherein;

one of Ring A and Ring B is a 5-membered.or 6-membered heterocyclic ring having one or two hetero atoms selected from nitrogen and sulfur atoms, in addition to carbon atoms, while the other is a benzene ring, and the Rings A and B may have one or two substituents selected from a halogen atom and an optionally halogenated $C_{1-4}$ alkyl group;

Ring C is a benzene ring optionally having from 1 to 3 substituents selected from a halogen atom, an optionally halogenated $C_{1-6}$ alkyl group (preferably, $C_{1-4}$ alkyl group) and an optionally halogenated $C_{1-6}$ alkoxy group (preferably, $C_{1-4}$ alkoxy group);
D that constitutes Ring Z is $-(CH_2)_m-$ (wherein m is an integer of from 1 to 7) or $-O-(CH_2)_p-$ (wherein p is an integer of from 1 to 7);
E that constitutes Ring Z is >C=O;

$$— X \overline{\quad\quad} \text{----------} Y <$$

is -CO-N< or -N=C<;
n is 1,
or pharmaceutically-acceptable salts thereof.

The above-mentioned "5-membered or 6-membered heterocyclic ring" includes, for example, pyridine, pyrazine, pyrrole, thiophene, thiazole, tetrahydropyrazine, piperidine, etc. Concretely, Ring A includes heterocyclic rings of the above-mentioned formula (A-5), etc., and Ring B includes benzene rings of the above-mentioned formulae (B-7) and (B-8), especially the above-mentioned formula (B-10), etc.

The above-mentioned "halogen atom" includes, for example, fluorine, chlorine and bromine atoms, etc.; the "optionally halogenated $C_{1-4}$ alkyl group" includes, for example, methyl, chloromethyl, difluoromethyl, trichloromethyl, trifluoromethyl, ethyl, 2-bromoethyl, 2,2,2-trifluoroethyl, perfluoroethyl, propyl, 3,3,3-trifluoropropyl, isopropyl, 2-trifluoromethylethyl, butyl, 4,4,4-trifluorobutyl, isobutyl, sec-butyl and tert-butyl groups, etc.; the "optionally halogenated $C_{1-6}$ alkyl group" includes pentyl and hexyl groups, etc., in addition to the above-mentioned alkyl groups and halogenated alkyl groups.

The "optionally halogenated $C_{1-4}$ alkoxy group" includes, for example, methoxy, difluoromethoxy, trifluoromethoxy, ethoxy, 2,2,2-trifluoroethoxy, perfluoroethoxy, propoxy, isopropoxy, butoxy, 4,4,4-trifluorobutoxy, isobutoxy, sec-butoxy and tert-butoxy groups, etc.; and the "optionally halogenated $C_{1-6}$ alkoxy group" includes pentyloxy and hexyloxy groups, etc., in addition to the above-mentioned alkoxy groups and halogenated alkoxy groups.

Of compounds of the above-mentioned general formula (I), especially those of the above-mentioned general formula (Ia), also preferred are (2) the following compounds or pharmaceutically-acceptable salts thereof.

Compounds wherein; Ring A is a 5-membered or 6-membered heterocyclic ring having one nitrogen atom or one sulfur atom, in addition to carbon atoms, for example, a heterocyclic ring of a formula (A-7):

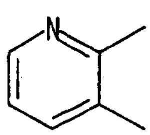 , 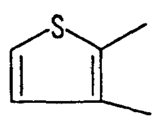 (A-7)

Ring B is a benzene ring optionally having 1 to 3 substituents selected from a halogen atom and an optionally halogenated $C_{1-4}$ alkyl group;
Ring C is a benzene ring optionally having 1 to 3 substituents selected from a halogen atom, an optionally halogenated $C_{1-4}$ alkyl group and an optionally halogenated $C_{1-4}$ alkoxy group;
D that constitutes Ring Z is $-(CH_2)_m-$ (wherein m is an integer of from 1 to 7) or $-O-(CH_2)_p-$ (wherein p is integer of from 1 to 7);
E that constitutes Ring Z is >C=O;

$$— X \overline{\quad\quad} \text{----------} Y<$$

is -CO-N<;
n is 1,
or pharmaceutically acceptable salts thereof.

As examples of the "halogen atom", the "optionally halogenated $C_{1-4}$ alkyl group" and the "optionally halogenated $C_{1-4}$ alkoxy group", referred to are the same atoms or groups as those above-mentioned compounds (1).

More preferably, compounds wherein; $R^a$ and $R^b$ are the same or different and each represent a hydrogen atom or a $C_1$ alkyl group optionally substituted by (1) $C_{1-6}$ alkoxy group, (2) $C_{1-6}$ alkylthio group, (3) amino group, (4)

$C_{1-7}$ acylainino group, (5) mono- or di-$C_{1-6}$ alkylamino group, (6) $C_{5-9}$ cyclic amino group (cyclic amino group optionally containing 1 to 3 hetero atom(s) of oxygen, sulfur atom, etc., in addition to nitrogen atom), (7) 5-membered or 6-membered cyclic amino group optionally substituted by $C_{1-6}$ alkyl group, (8) $C_{1-6}$ alkylsulfonylamino group or (9) $C_{1-6}$ alkylcarbonyloxy group; or
$R^a$ and $R^b$ are bonded to each other to form pyridine ring which is optionally substituted by 1 to 3 substituents selected from a halogen atom and a $C_{1-4}$ alkyl group;

Ring B is a benzene ring optionally having 1 to 3 substituents selected from (1) a halogen atom, (2) an optionally halogenated $C_{1-4}$ alkyl group and (3) an optionally halogenated $C_{1-4}$ alkoxy group;
Ring C is a benzene ring optionally having 1 to 3 substituents selected from (1) a halogen atom, (2) an optionally halogenated $C_{1-4}$ alkyl group, (3) an optionally halogenated $C_{1-4}$ alkoxy group, (4) an amino group optionally substituted by $C_{1-4}$ alkyl group, (5) a $C_{1-3}$ acyloxy group and (6) a hydroxy group;
Ring Z is a 5-membered to 10-membered nitrogen containing heterocyclic ring optionally having an oxo group and optionally substituted $C_{1-4}$ alkyl group or a hydroxy group;

— X ---------- Y<

is -CO-N< or -N=C<;
and n is 1, or salts thereof.

Preferred compounds of formulae (I) and (Ia) include, for example, compounds of the following general formula:

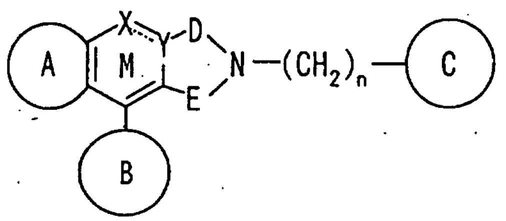

wherein D and E represent alkylene groups optionally having an oxo group and the other symbols are as defined above, or salts thereof.
Preferably, D and E represent, independently, a $C_{1-3}$ alkylene group optionally substituted by one oxo group.
More preferred compounds of formulae (I) and (Ia) include, for example, compounds of the following general formula:

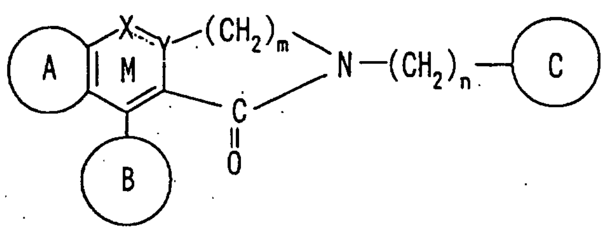

wherein m represents an integer of from 1 to 7, and the other symbols are as defined above or salts thereof.
m is preferably an integer of from 2 to 5.
In the above-mentioned formulae, preferably $R^a$ and $R^b$ are the same or different and each represent a hydrogen atom or a substituent selected from the group consisting of

(1) a halogen atom,
(2) a $C_{1-6}$ alkyl group optionally having from 1 to 5 substituents selected from the group consisting of

(a) a hydroxy group,
(b) a $C_{1-6}$ alkoxy group,

(c) a $C_{1-6}$ alkylthio group,
(d) an amino group,
(e) a $C_{1-7}$ acylamino group,
(f) a mono- or di-$C_{1-6}$ alkylamino group,
(g) a mono- or di-$C_{3-8}$ cycloalkylamino group,
(h) a 5-membered to 9-membered cyclic amino group which may have 1 to 3 hetero atoms selected from the group consisting of oxygen and sulfur atoms in addition to nitrogen atom and which may be substituted by $C_{1-6}$ alkyl group,
(i) a $C_{1-4}$ alkylsulfonylamino group,
(j) a $C_{1-6}$ alkyl-carbonyloxy group and
(k) a halogen atom,

(3) a 5-membered to 9-membered (preferably 6-membered) cyclic amino group which may have 1 to 3 hetero atoms (preferably 1 or 2) selected from the group consisting of oxygen and sulfur atoms, in addition to nitrogen atom and which may be substituted by $C_{1-6}$ alkyl group,
(4) a carboxyl group,
(5) carbamoyl group,
(6) a mono- or di-$C_{1-6}$ alkylcarbamoyl group; or

$R^a$ and $R^b$ are bonded to each other to form Ring A, and the Ring A is a 5-membered to 9-membered aromatic heterocyclic ring having from 1 to 3 hetero atoms selected from the group consisting of nitrogen, sulfur and oxygen atoms, in addition to carbon atoms (preferably pyridine ring), which may be substituted by $C_{1-6}$ alkyl group;
the Ring B is a $C_{6-10}$ aryl group (preferably benzene ring) which may be substituted by substituents selected from the group consisting of (i) a $C_{1-6}$ alkyl group optionally substituted by a hydroxy group, (ii) a $C_{1-6}$ alkyl-carbonyl group (including formyl) and (iii) a carboxyl group;
the Ring C is a $C_{6-10}$ aryl group (preferably benzene ring) which may be substituted by 1 to 3 substituents selected from the group consisting of (i) a halogen atom, (ii) optionally halogenated $C_{1-10}$ alkyl group and (iii) $C_{1-10}$ alkoxy group;
the Ring Z is a 5-membered to 12-membered heterocyclic ring optionally having at least one hetero atom selected from the group consisting of nitrogen, oxygen and sulfur atoms, in addition to Y and nitrogen atom, which may be substituted by 1 to 3 substituents selected from the group consisting of (i) a $C_{1-6}$ alkyl group, (ii) a hydroxy group and (iii) oxo group.

Where the compounds of (I) and (Ia) may form salts and used in medicines, it is preferable that the salts are pharmaceutically-acceptable salts.
Examples of such pharmaceutically-acceptable salts include salts with inorganic acids, such as hydrochloric acid, sulfuric acid, phosphoric acid, diphosphoric acid, hydrobromic acid, nitric acid, etc., or salts with organic acids, such as acetic acid, malic acid, maleic acid, fumaric acid, tartaric acid, succinic acid, citric acid, lactic acid, methanesulfonic acid, p-toluenesulfonic acid, palmitic acid, salicylic acid, stearic acid, etc.
The compounds of (I) and (Ia) or salts thereof, which are used in the present invention, include stereoisomers such as cis- and trans-isomers, etc., racemates, as well as optically-active forms such as R-forms, S-forms, etc. Depending on the size of Ring Z, conformation-dependent isomers may be occurred and the compounds of (I) and (Ia) or salts thereof may include these isomers.
Preferable compounds included in compounds (I) and (Ia) are shown in the following.

(1) 7-[3,5-bis(trifluoromethyl)benzyl]-6,7,8,9-tetrahydro-5-(4-methylphenyl)-6,11-dioxo-11H-pyrazino[2,1-g][1,7]naphthyridine (hereinafter sometimes abbreviated as compound No. 1)
(2) 7-[3,5-bis(trifluoromethyl)benzyl]-6,7,8,9,10,12-hexahydro-5-(4-methyl)phenyl-6,12-dioxo-[1,4]diazepino[2,1-g][1,7]naphthyridine (hereinafter sometimes abbreviated as compound No. 2)
(3) 7-[3,5-bis(trifluoromethyl)benzyl]-6,7,8,9,10,11-hexahydro-5-(4-methylphenyl)-6,13-dioxo-13H-[1,4]diazocino[2,1-g][1,7]naphthyridine
(4) 6,7,8,9,10,12-hexahydro-7-(2-methoxybenzyl)-5-(4-methylphenyl)-6,12-dioxo-[1,4]diazepino[2,1-g][1,7]naphthyridine
(5) 6,7,8,9,10,11-hexahydro-7-(2-methoxybenzyl)-5-(4-methylphenyl)-6,13-dioxo-13H-[1,4]diazocino[2,1-g][1,7]naphthyridine
(6) 7-[3,5-bis(trifluoromethyl)benzyl]-6,7,8,9,10,11,12,14-octahydro-5-(4-methylphenyl)-6,14-dioxo[1,4]diazonino[2,1-g][1,7]naphthyridine

(7) 7-[3,5-bis(trifluoromethyl)benzyl]-6,7,8,9,10,12-hexahydro-6,12-dioxo-5-phenyl[1,4]diazepino[2,1-g][1,7]naphthyridine
(8) 7-[3,5-bis(trifluoromethyl)benzyl]-6,7,8,9,10,11-hexahydro-6,13-dioxo-5-phenyl-13H-[1,4]diazocino[2,1-g][1,7]naphthyridine
(9) 7-[3,5-bis(trifluoromethyl)benzyl]-6,7,8,9,10,11-hexahydro-5-(4-methylphenyl)-6,13-dioxo-13H-[1,4]diazocino[1,2-b][2,7]naphthyridine
(10) 7-[3,5-bis(trifluoromethyl)benzyl]-1,2,3,4,6,7,8,9,10,11-decahydro-2-methyl-5-(4-methylphenyl)-6,13-dioxo-13H-[1,4]diazocino[1,2-b][2,7]naphthyridine
(11)4-[3,5-bis(trifluoromethyl)benzyl]-2,3,4,5-tetrahydro-5-oxo-6-phenylpyrido[3,2-f][1,4]oxazepine
(12) (9R)-7-[3,5-bis(trifluoromethyl)benzyl]-6,7,8,9,10,12-hexahydro-9-methyl-5-(4-methylphenyl)-6,12-dioxo[1,4]diazepino[2,1-g][1,7]naphthyridine
(13) (9S)-7-[3,5-bis(trifluoromethyl)benzyl]-6,7,8,9,10,12-hexahydro-9-methyl-6,12-dioxo-5-phenyl[1,4]diazepino[2,1-g][1,7]naphthyridine
(14) (9S)-7-[3,5-bis(trifluoromethyl)benzyl]-6,7,8,9,10,12-hexahydro-9-methyl-5-(4-methylphenyl)-6,12-dioxo[1,4]diazepino[2,1-g][1,7]naphthyridine
(15) (±)-7-[3,5-bis(trifluoromethyl)benzyl]-6,7,8,9,10,11-hexahydro-9-methyl-6,13-dioxo-5-phenyl-13H-[1,4]diazocino[2,1-g][1,7]naphthyridine
(16) (±)-7-[3,5-bis(trifluoromethyl)benzyl]-6,7,8,9,10,11-hexahydro-9-methyl-5-(4-methylphenyl)-6,13-dioxo-13H-[1,4]diazocino[2,1-g][1,7]naphthyridine
(17) (9R)-7-[3,5-bis(trifluoromethyl)benzyl]-6,7,8,9,10,11-hexahydro-9-methyl-6,13-dioxo-5-phenyl-13H-[1,4]diazocino[2,1-g][1,7]naphthyridine
(18) (9R)-7-[3,5-bis(trifluoromethyl)benzyl]-6,7,8,9,10,11-hexahydro-9-methyl-5-(4-methylphenyl)-6,13-dioxo-13H-[1,4]diazocino[2,1-g][1,7]naphthyridine (hereinafter sometimes abbreviated as compound No. 3)
(19) (9S)-7-[3,5-bis(trifluoromethyl)benzyl]-6,7,8,9,10,11-hexahydro-9-methyl-6,13-dioxo-5-phenyl-13H-[1,4]diazocino[2,1-g][1,7]naphthyridine
(20) (9S)-7-[3,5-bis(trifluoromethyl)benzyl]-6,7,8,9,10,11-hexahydro-9-methyl-5-(4-methylphenyl)-6,13-dioxo-13H-[1,4]diazocino[2,1-g][1,7]naphthyridine
(21) 4-[3,5-bis(trifluoromethyl)benzyl]-2,3,4,5-tetrahydro-5-oxo-6-phenyl-1H-pyrido[2,3-e][1,4]diazepine
(22) 5-[3,5-bis(trifluoromethyl)benzyl]-2,3,4,5-tetrahydro-6-oxo-7-phenyl-6H-pyrido[2,3-b][1,5]oxazocine
(23) 4-[3,5-bis(trifluoromethyl)benzyl]-2,3,4,5-tetrahydro-7-methyl-5-oxo-6-phenylpyrido[3,2-f][1,4]oxazepine
(24) 5-[3,5-bis(trifluoromethyl)benzyl]-2,3,4,5-tetrahydro-8-methyl-6-oxo-7-phenyl-6H-pyrido[2,3-b][1,5]oxazocine
(25) (±)-7-[3,5-bis(trifluoromethyl)benzyl]-6,7,8,9,10,12-hexahydro-9-hydroxy-5-(4-methylphenyl)-6,12-dioxo[1,4]diazepino[2,1-g][1,7]naphthyridine
(26) 7-benzyl-6,7,8,9,10,12-hexahydro-6,12-dioxo-5-phenyl[1,4]diazepino[2,1-g][1,7]naphthyridine
(27) 7-benzyl-6,7,8,9,10,11-hexahydro-6,13-dioxo-5-phenyl-13H-[1,4]diazocino[2,1-g][1,7]naphthyridine
(28) 7-benzyl-6,7,8,9,10,11,12,14-octahydro-6,14-dioxo-5-phenyl[1,4]diazonino[2,1-g][1,7]naphthyridine
(29) 7-(3,4-dichlorobenzyl)-6,7,8,9,10,12-hexahydro-6,12-dioxo-5-phenyl[1,4]diazepino[2,1-g][1,7]naphthyridine
(30) 7-(3,4-dichlorobenzyl)-6,7,8,9,10,11-hexahydro-6,13-dioxo-5-phenyl-13H-[1,4]diazocino[2,1-g][1,7]naphthyridine
(31) (S)-5-[3,5-bis(trifluoromethyl)benzyl]-2,3,4,5-tetrahydro-3,8-dimethyl-6-oxo-7-phenyl-6H-pyrido[2,3-b][1,5]oxazocine
(32) (R)-5-[3,5-bis(trifluoromethyl)benzyl]-2,3,4,5-tetrahydro-3,8-dimethyl-6-oxo-7-phenyl-6H-pyrido[2,3-b][1,5]oxazocine
(33) 4-[3,5-bis(trifluoromethyl)benzyl]-2,3,4,5-tetrahydro-8-methyl-5-oxo-6-phenylpyrido[3,2-f][1,4]oxazepine
(34) 5-[3,5-bis(trifluoromethyl)benzyl]-2,3,4,5-tetrahydro-9-methyl-6-oxo-7-phenyl-6H-pyrido[2,3-b][1,5]oxazocine
(35) 4-[3,5-bis(trifluoromethyl)benzyl]-2,3,4,5-tetrahydro-8-methyl-5-oxo-6-phenylpyrido[3,2-f][1,4]oxazepine 9-oxide
(36) 5-[3,5-bis(trifluoromethyl)benzyl]-2,3,4,5-tetrahydro-9-methyl-6-oxo-7-phenyl-6H-pyrido[2,3-b][1,5]oxazocine 10-oxide
(37) 8-acetoxymethyl-4-[3,5-bis(trifluoromethyl)benzyl]-2,3,4,5-tetrahydro-5-oxo-6-phenylpyrido-[3,2-f][1,4]oxazepine
(38) 9-acetoxymethyl-5-[3,5-bis(trifluoromethyl)benzyl]-2,3,4,5-tetrahydro-6-oxo-7-phenyl-6H-pyrido[2,3-b][1,5]oxazocine
(39) 4-[3,5-bis(trifluoromethyl)benzyl]-8-chloromethyl-2,3,4,5-tetrahydro-5-oxo-6-phenylpyrido[3,2-f][1,4]oxazepine
(40) 4-[3,5-bis(trifluoromethyl)benzyl]-2,3,4,5-tetrahydro-8-methoxymethyl-5-oxo-6-phenylpyrido[3,2-f][1,4]oxazepine
(41) 4-[3,5-bis(trifluoromethyl)benzyl]-2,3,4,5-tetrahydro-8-(2-methylethyl)-5-oxo-6-phenylpyrido[3,2-f][1,4]oxazepine
(42) 4-[3,5-bis(trifluoromethyl)benzyl]-2,3,4,5-tetrahydro-8-methylthiomethyl-5-oxo-6-phenylpyrido[3,2-f][1,4]oxazepine
(43) 8-aminomethyl-4-[3,5-bis(trifluoromethyl)benzyl]-2,3,4,5-tetrahydro-5-oxo-6-phenylpyrido[3,2-f][1,4]oxazepine
(44) 4-[3,5-bis(trifluoromethyl)benzyl]-2,3,4,5-tetrahydro-8-methylaminomethyl-5-oxo-6-phenylpyrido[3,2-f][1,4]oxazepine
(45) 4-[3,5-bis(trifluoromethyl)benzyl]-8-dimethylaminomethyl-2,3,4,5-tetrahydro-5-oxo-6-phenylpyrido[3,2-f][1,4]oxazepine
(46) 4-[3,5-bis(trifluoromethyl)benzyl]-8-cyclopropylaminomethyl-2,3,4,5-tetrahydro-5-oxo-6-phenylpyrido[3,2-f][1,4]oxazepine
(47) 4-[3,5-bis(trifluoromethyl)benzyl]-2,3,4,5-tetrahydro-8-(N-methylpiperazinomethyl)-5-oxo-6-phenylpyrido[3,2-f][1,4]oxazepine
(48) 8-acetylaminomethyl-4-[3,5-bis(trifluoromethyl)benzyl]-2,3,4,5-tetrahydro-5-oxo-6-phenylpyrido[3,2-f][1,4]oxazepine
(49) 4-[3,5-bis(trifluoromethyl)benzyl]-2,3,4,5-tetrahydro-8-methanesulfonylaminomethyl-5-oxo-6-phenylpyrido[3,2-f][1,4]oxazepine
(50) 6-[3,5-bis(trifluoromethyl)benzyl]-5,6,7,8-hexahydro-3,9-dimethyl-5,10-dioxo-4-phenylpyrido[2,3-f][1,4]diazocine
(51) 6-[3,5-bis(trifluoromethyl)benzyl]-5,6,7,8,9,10-hexahydro-9-methyl-5,10-dioxo-4-phenylpyrido[2,3-f][1,4]diazocine
(52) 6-benzyl-5,6,7,8,9,10-hexahydro-3,9-dimethyl-5,10-dioxo-4-phenylpyrido[2,3-f][1,4]diazocine
(53) 6-[3,5-bis(trifluoromethyl)benzyl]-9-ethyl-5,6,7,8,9,10-hexahydro-3-methyl-5,10-dioxo-4-phenylpyrido[2,3-f][1,4]diazocine
(54) 6-[3,5-bis(trifluoromethyl)benzyl]-6,7,8,9,10,11-hexahydro-3,10-dimethyl-5,11-dioxo-4-phenyl-5H-pyrido[2,3-g][1,5]diazonine
(55) 4-[3,5-bis(trifluoromethyl)benzyl]-8-hydroxymethyl-2,3,4,5-tetrahydro-5-oxo-6-phenylpyrido[3,2-f][1,4]oxazepine
(56) 4-[3,5-bis(trifluoromethyl)benzyl]-2,3,4,5-tetrahydro-5-oxo-6-phenylpyrido[3,2-f][1,4]oxazepine-8-carboxylic acid
(57) 4-[3,5-bis(trifluoromethyl)benzyl]-2,3,4,5-tetrahydro-5-oxo-6-phenylpyrido[3,2-f][1,4]oxazepine-8-carboxamide
(58) 4-[3,5-bis(trifluoromethyl)benzyl]-N-methyl-2,3,4,5-tetrahydro-5-oxo-6-phenylpyrido[3,2-f][1,4]oxazepine-8-carboxamide
(59) 4-[3,5-bis(trifluoromethyl)benzyl]-N,N-dimethyl-2,3,4,5-tetrahydro-5-oxo-6-phenylpyrido[3,2-f][1,4]oxazepine-8-carboxamide
(60) 4-[3,5-bis(trifluoromethyl)benzyl]-N-n-butyl-2,3,4,5-tetrahydro-5-oxo-6-phenylpyrido[3,2-f][1,4]oxazepine-8-carboxamide
(61) 4-[3,5-bis(trifluoromethyl)benzyl]-2,3,4,5-tetrahydro-5-oxo-6-phenyl-8-piperidinocarbonylpyrido[3,2-f][1,4]oxazepine
(62) 4-[3,5-bis(trifluoromethyl)benzyl]-2,3,4,5-tetrahydro-8-morpholinocarbonyl-5-oxo-6-phenylpyrido[3,2-f][1,4]oxazepine
(63) 4-[3,5-bis(trifluoromethyl)benzyl]-2,3,4,5-tetrahydro-8-[1-(4-methylpiperazinyl)carbonyl]-5-oxo-6-phenylpyrido[3,2-f][1,4]oxazepine
(64) 2,3,4,5-tetrahydro-5-oxo-6-phenyl-4-(3,4,5-trimethoxybenzyl)pyrido[3,2-f][1,4]oxazepine
(65) 4-(3,4-dichlorobenzyl)-2,3,4,5-tetrahydro-5-oxo-6-phenylpyrido[3,2-f][1,4]oxazepine
(66) 4-(3,4-dimethoxybenzyl)-2,3,4,5-tetrahydro-5-oxo-6-phenylpyrido[3,2-f][1,4]oxazepine
(67) 4-benzyl-2,3,4,5-tetrahydro-5-oxo-6-phenylpyrido[3,2-f][1,4]oxazepine
(68) 2,3,4,5-tetrahydro-6-oxo-7-phenyl-5-(3,4,5-trimethoxybenzyl)-6H-pyrido[2,3-b][1,5]oxazocine
(69) (S)-5-benzyl-2,3,4,5-tetrahydro-3-methyl-6-oxo-7-phenyl-6H-pyrido[2,3-b][1,5]oxazocine
(70) (R)-5-benzyl-2,3,4,5-tetrahydro-3-methyl-6-oxo-7-phenyl-6H-pyrido[2,3-b][1,5]oxazocine
(71) (S)-5-[3,5-bis(trifluoromethyl)benzyl]-2,3,4,5-tetrahydro-3-methyl-6-oxo-7-phenyl-6H-pyrido[2,3-b][1,5]oxazocine
(72) (R)-5-[3,5-bis(trifluoromethyl)benzyl]-2,3,4,5-tetrahydro-3-methyl-6-oxo-7-phenyl-6H-pyrido[2,3-b][1,5]oxazocine
(73) 7-benzyl-6,7,8,9,10,11-hexahydro-5-(4-methylphenyl)-6,13-dioxo-13H-[1,4]diazocino[2,1-g][1,7]naphthyridine
(74) (9R)-7-benzyl-6,7,8,9,10,11-hexahydro-9-methyl-5-(4-methylphenyl)-6,13-dioxo-13H-[1,4]diazocino[2,1-g][1,7]naphthyridine
(75) (9S)-7-benzyl-6,7,8,9,10,11-hexahydro-9-methyl-5-(4-methylphenyl)-6,13-dioxo-13H-[1,4]diazocino[2,1-g][1,7]naphthyridine
(76) (9R)-6,7,8,9,10,11-hexahydro-9-methyl-5-(4-methylphenyl)-6,13-dioxo-7-(3,4,5-trimethoxybenzyl)-13H-[1,4]diazocino[2,1-g][1,7]naphthyridine
(77) (9S)-6,7,8,9,10,11-hexahydro-9-methyl-5-(4-methylphenyl)-6,13-dioxo-7-(3,4,5-trimethoxybenzyl)-13H-[1,4]diazocino[2,1-g][1,7]naphthyridine
(78) (9R)-7-(3,5-dimethoxybenzyl)-6,7,8,9,10,11-hexahydro-9-methyl-5-(4-methylphenyl)-6,13-dioxo-13H-[1,4]diazocino[2,1-g][1,7]naphthyridine
(79) 4-benzyl-2,3,4,5-tetrahydro-5-oxo-6-(4-methylphenyl)pyrido[3,2-f][1,4]oxazepine
(80) 4-[3,5-bis(trifluoromethyl)benzyl]-2,3,4,5-tetrahydro-5-oxo-6-(4-methylphenyl)pyrido[3,2-f][1,4]oxazepine
(81) (S)-5-benzyl-2,3,4,5-tetrahydro-3-methyl-7-(4-methylphenyl)-6-oxo-6H-pyrido[2,3-b][1,5]oxazocine
(82) (S)-5-[3,5-bis(trifluoromethyl)benzyl-2,3,4,5-tetrahydro-3-methyl-7-(4-methylphenyl)-6-oxo-6H-pyrido[2,3-b][1,5]oxazocine
(83) (9R)-7-[3,5-bis(trifluoromethyl)benzyl]-6,7,8,9,10,11-hexahydro-5-(4-hydroxymethylphenyl)-9-methyl-6,13-dioxo-13H-[1,4]diazocino[2,1-g][1,7]naphthyridine
(84) (9R)-7-[3,5-bis(trifluoromethyl)benzyl]-5-(4-formylphenyl)-6,7,8,9,10,11-hexahydro-9-methyl-6,13-dioxo-13H-[1,4]diazocino[2,1-g][1,7]naphthyridine
(85) (9R)-7-[3,5-bis(trifluoromethyl)benzyl]-5-(4-formylphenyl)-6,7,8,9,10,11-hexahydro-9-methyl-6,13-dioxo-13H-[1,4]diazocino[2,1-g][1,7]naphthyridine
(86) (9R)-7-[3,5-bis(trifluoromethyl)benzyl]-5-(4-carboxyphenyl)-6,7,8,9,10,11-hexahydro-9-methyl-6,13-dioxo-13H-[1,4]diazocino[2,1-g][1,7]naphthyridine
(87) (9R)-7-[3,5-bis(trifluoromethyl)benzyl]-6,7,8,9,10,11-hexahydro-9-methyl-5-(4-methylphenyl)-6,13-dioxo-13H-[1,4]diazocino[2,1-g][1,7]naphthyridine N-oxide
(88) (9R)-7-[3,5-bis(trifluoromethyl)benzyl]-6,7,8,9,10,11-hexahydro-5-(4-hydroxymethylphenyl)-9-methyl-6,13-dioxo-13H-[1,4]diazocino[2,1-g][1,7]naphthyridine N-oxide
(89) (9R)-7-[3,5-bis(trifluoromethyl)benzyl]-5-(4-carboxyphenyl)-6,7,8,9,10,11-hexahydro-9-methyl-6,13-dioxo-13H-[1,4]diazocino[2,1-g][1,7]naphthyridine N-oxide
(90) (9S)-[10,10,11,11-$^2H_4$]-7-[3,5-bis(trifluoromethyl)benzyl]-8,9,10,11-tetrahydro-5-(4-hydroxymethylphenyl)-9-methyl-7H-[1,4]diazocino[2,1-g][1,7]naphthyridine-6,13-dione ($d_4$ form of the compound of the above-mentioned (83))
(91) (9S)-[10,10,11,11-$^2H_4$]-7-[3,5-bis(trifluoromethyl)benzyl]-5-(4-formylphenyl)-8,9,10,11-tetrahydro-9-methyl-7H-[1,4]diazocino[2,1-g][1,7]naphthyridine-6,13-dione ($d_4$ form of the compound of the above-mentioned (84))
(92) (9S)-[10,10,11,11-$^2H_4$]-7-[3,5-bis(trifluoromethyl)benzyl]-5-(4-carboxyphenyl)-8,9,10,11-tetrahydro-9-methyl-7H-[1,4]diazocino[2,1-g][1,7]naphthyridine-6,13-dione ($d_4$ form of the compound of the above-mentioned (86))
(93) (9R)-7-[3,5-di(benzyloxy)benzyl]-6,7,8,9,10,11-hexahydro-9-methyl-5-(4-methylphenyl)-6,13-dioxo-13H-[1,4]diazocino[2,1-g][1,7]naphthyridine
(94) (9R)-7-(3,5-dihydroxybenzyl)-6,7,8,9,10,11-hexahydro-9-methyl-5-(4-methylphenyl)-6,13-dioxo-13H-[1,4]diazocino[2,1-g][1,7]naphthyridine
(95) (9R)-7-(3,5-diethoxybenzyl)-6,7,8,9,10,11-hexahydro-9-methyl-5-(4-methylphenyl)-6,13-dioxo-13H-[1,4]diazocino[2,1-g][1,7]naphthyridine
(96) (9R)-7-[3,5-di(1-methylethyloxy)benzyl]-6,7,8,9,10,11-hexahydro-9-methyl-5-(4-methylphenyl)-6,13-dioxo-13H-[1,4]diazocino[2,1-g][1,7]naphthyridine
(97) (9R)-7-(3,5-dimethoxybenzyl)-6,7,8,9,10,11-hexahydro-9-methyl-6,13-dioxo-5-phenyl-13H-[1,4]diazocino[2,1-g][1,7]naphthyridine
(98) (9R)-7-[3,5-bis(trifluoromethyl)benzyl]-5-(4-chlorophenyl)-6,7,8,9,10,11-hexahydro-9-methyl-6,13-dioxo-13H-[1,4]diazocino[2,1-g][1,7]naphthyridine
(99) (9R)-7-[3,5-bis(trifluoromethyl)benzyl]-5-(3,4-dichlorophenyl)-6,7,8,9,10,11-hexahydro-9-methyl-6,13-dioxo-13H-[1,4]diazocino[2,1-g][1,7]naphthyridine
(100) (9R)-7-(3,5-dimethoxybenzyl)-5-(3,4-dichlorophenyl)-6,7,8,9,10,11-hexahydro-9-methyl-6,13-dioxo-13H-[1,4]diazocino[2,1-g][1,7]naphthyridine
(101) (9R)-7-(3,5-dimethylbenzyl)-6,7,8,9,10,11-hexahydro-9-methyl-5-(4-methylphenyl)-6,13-dioxo-13H-[1,4]diazocino[2,1-g][1,7]naphthyridine
(102) (9R)-7-(3,5-dichlorobenzyl)-6,7,8,9,10,11-hexahydro-9-methyl-5-(4-methylphenyl)-6,13-dioxo-13H-[1,4]diazocino[2,1-g][1,7]naphthyridine
(103) (9R)-5-(3,4-dichlorophenyl)-7-(3,5-dimethylbenzyl)-6,7,8,9,10,11-hexahydro-9-methyl-6,13-dioxo-13H-[1,4]diazocino[2,1-g][1,7]naphthyridine
(104) (9R)-7-(3,5-dimethoxybenzyl)-5-(4-fluorophenyl)-6,7,8,9,10,11-hexahydro-9-methyl-6,13-dioxo-13H-[1,4]diazocino[2,1-g][1,7]naphthyridine
(105) (9R)-5-(4-chlorophenyl)-7-(3,5-dimethoxybenzyl)-6,7,8,9,10,11-hexahydro-9-methyl-6,13-dioxo-13H-[1,4]diazocino[2,1-g][1,7]naphthyridine
(106) (9R)-7-[3,5-bis(trifluoromethyl)benzyl]-5-(4-fluorophenyl)-6,7,8,9,10,11-hexahydro-9-methyl-6,13-dioxo-13H-[1,4]diazocino[2,1-g][1,7]naphthyridine
(107) (±)-7-[3,5-bis(trifluoromethyl)benzyl]-9-ethyl-6,7,8,9,10,11-hexahydro-5-(4-methylphenyl)-6,13-dioxo-13H-[1,4]diazocino[2,1-g][1,7]naphthyridine
(108) (±)-7-[3,5-bis(trifluoromethyl)benzyl]-6,7,8,9,10,11-hexahydro-9-(1-methylethyl)-5-(4-methylphenyl)-6,13-dioxo-13H-[1,4]diazocino[2,1-g][1,7]naphthyridine
(109) (±)-5-(3,4-dichlorophenyl)-7-(3,5-dimethoxybenzyl)-9-ethyl-6,7,8,9,10,11-hexahydro-6,13-dioxo-13H-[1,4]diazocino[2,1-g][1,7]naphthyridine
(110) (±)-5-(3,4-dichlorophenyl)-7-(3,5-dimethoxybenzyl)-6,7,8,9,10,11-hexahydro-9-(1-methylethyl)-6,13-dioxo-13H-[1,4]diazocino[2,1-g][1,7]naphthyridine

**Production method of compound or a salt thereof**

The compounds (I) and (Ia) and salts thereof can be produced according to the methods described in JP-A-9-263585 and JP-A-10-109989.

The compounds of the above-mentioned (1)-(82) can be produced based on the description of Examples of JP-A-9-263585. The compounds of the above-mentioned (83)-(92) can be produced based on the description of Examples of JP-A-10-109989. The compounds of the above-mentioned (93)-(110) can be produced according to the description of Examples of JP-A-9-263585 or JP-A-10-109989.

These NK-1 receptor antagonists may form a salt and examples of the salt of the NK-1 receptor antagonist include metal salt, ammonium salt, salt with organic base, salt with inorganic acid, salt with organic acid, salt with basic or acidic amino acid and the like.

Preferable examples of the metal salt include alkali metal salts such as sodium salt, potassium salt and the like; alkaline earth metal salts such as calcium salt, magnesium salt, barium salt and the like; aluminum salt and the like.

Preferable examples of the salt with organic base include salts with trimethylamine, triethylamine, pyridine, picoline, 2,6-lutidine, ethanolamine, diethanolamine, triethanolamine, cyclohexylamine, dicyclohexylamine, N,N'-dibenzylethylenediamine and the like.

Preferable examples of the salt with inorganic acid include salts with hydrochloric acid, hydrobromic acid, nitric acid, sulfuric acid, phosphoric acid and the like. Preferable examples of the salt with organic acid include salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid and the like.

Preferable examples of the salt with basic amino acid include salts with arginine, lysin, ornithine and the like, and preferable examples of salts with acidic amino acid include salts with aspartic acid, glutamic acid and the like.

Of these, pharmaceutically acceptable salts are preferable. When a compound has an acidic functional group, inorganic salts such as alkali metal salt (e.g., sodium salt, potassium salt and the like), alkaline earth metal salt (e.g., calcium.salt, magnesium salt, barium salt and the like) and the like, ammonium salt and the like; when a compound has a basic functional group, salts with inorganic acid, such as hydrochloric acid, hydrobromic acid, nitric acid, sulfuric acid, phosphoric acid and the like, or salts with organic acid such as acetic acid, phthalic acid, fumaric acid, oxalic acid, tartaric acid, maleic acid, citric acid, succinic acid, methanesulfonic acid, p-toluenesulfonic acid and the like can be exemplified.

Moreover, the NK-1 receptor antagonist may be a prodrug. Concretely, the prodrug means a compound converted to an NK-1 receptor antagonist under the physiological conditions or by a reaction due to an enzyme, gastric acid, etc. in the body, that is, a compound converted to an NK-1 receptor antagonist by oxidation, reduction, hydrolysis and the like according to an enzyme; a compound converted to an NK-1 receptor antagonist by hydrolysis according to gastric acid and the like.

Examples of the prodrug of the NK-1 receptor antagonist include compounds wherein amino groups of the NK-1 receptor antagonist are substituted with acyl, alkyl, phosphoric acid, etc. (e.g., compounds wherein amino groups of the NK-1 receptor antagonist are substituted with eicosanoyl, alanyl, pentylaminocarbonyl, (5-methyl-2-oxo-1,3-dioxolen-4-yl)methoxycarbonyl, tetrahydrofuranyl, pyrrolidylmethyl, pivaloyloxymethyl, tert-butyl, etc.); compounds wherein hydroxy groups of the NK-1 receptor antagonist are substituted with acyl, alkyl, phosphoric acid, boric acid, etc. (e.g., compounds wherein hydroxy groups of the NK-1 receptor antagonist are substituted with acetyl, palmitoyl, propanoyl, pivaloyl, succinyl, fumaryl, alanyl, dimethylaminomethylcarbonyl, etc.); compounds wherein carboxyl groups of the NK-1 receptor antagonist are modified with ester, amide, etc. (e.g., compounds wherein carboxyl groups of the

NK-1 receptor antagonist are modified with ethyl ester, phenyl ester, carboxymethyl ester, dimethylaminomethyl ester, pivaloyloxymethyl ester, ethoxycarbonyloxyethyl ester, phthalidyl ester, (5-methyl-2-oxo-1,3-dioxolen-4-yl)methyl ester, cyclohexyloxycarbonylethyl ester, methyl amide, etc.); etc. These prodrugs can be produced by per se known method from the NK-1 receptor antagonist.

The prodrug of the NK-1 receptor antagonist may be a compound which is converted to an NK-1 receptor antagonist under the physiological conditions as described in "Pharmaceutical Research and Development", Vol. 7 (Drug Design), pages 163-198 published in 1990 by Hirokawa Publishing Co. (Tokyo, Japan).

The present invention further provides novel use of the NK-1 receptor antagonist. That is, the present invention containing an NK-1 receptor antagonist is useful as an agent for the prophylaxis or treatment of depression accompanied by urinary frequency, urinary incontinence and/or irritable bowel syndrome, an agent for the prophylaxis or treatment of mood disorders of patients with urinary frequency and urinary incontinence, and a circadian rhythm controller for the hypothalamic endocrine system for mammals (e.g., mouse, rat, hamster, rabbit, cat, dog, bovine, sheep, monkey, human and the like). There is a report that almost 40% of the patients with urinary incontinence and urinary frequency suffer from propensity toward depression or a mood disorder similar to depression (e.g., anorexia, overeating, insomnia, hypersomnia, goneness or fatigue,. decline in self-esteem, drop in concentration power or indecisiveness, feelings of despair, irrits, nausea, throwing tantrums and the like). The present invention containing the NK-1 receptor antagonist provides an agent for the prophylaxis or treatment of depression, depression accompanied by urinary frequency, urinary incontinence and/or irritable bowel syndrome and a mood disorder of patients with urinary incontinence and urinary frequency, which is associated with anxiety disorder (agoraphobia, social phobia, post-traumatic stress disorder, acute stress disorder, generalized anxiety disorder, unidentifiable anxiety disorder and the like).

The NK-1 receptor antagonist can control the circadian rhythm of the hypothalamic endocrine system (e.g., secretion of vasopressin, VIP (vasoactive intestinal polypeptide), GRP (gastrin-releasing peptide) and the like).

Particularly, the NK-1 receptor antagonist can bring the night state of circadian rhythm of the hypothalamic endocrine system to a normal state. For example, when the secretion amount of vasopressin, VIP and GRP at night in human having disturbed circadian rhythm is small, the above-mentioned NK-1 receptor antagonist can make the living organism to a night type and normalize the secretion of vasopressin, VIP and GRP during night. Therefore, it exerts an extremely superior prophylactic and therapeutic effect on urinary frequency and urinary incontinence during night.

Thus, the present invention containing the NK-1 receptor antagonist is useful as a circadian rhythm secretion controller of the hypothalamic endocrine system in mammals (e.g., mouse, rat, hamster, rabbit, cat, dog, bovine, sheep, monkey, human and the like), and is further useful as an agent for the prophylaxis or therapy of urinary frequency and urinary incontinence during night.

The NK-1 receptor antagonist used here shows low toxicity and is safe.

Specific examples of the NK-1 receptor antagonist usable for the use of the above-mentioned NK-1 receptor antagonist as a novel pharmaceutical agent include those mentioned above.

More specifically, the NK-1 receptor antagonist to be contained in the above-mentioned various pharmaceutical agents is, for example, the above-mentioned compound (I), (Ia) or a salt thereof or a prodrug thereof.

When the above-mentioned NK-1 receptor antagonist and the NK-1 receptor antagonist having particular properties are used as the various pharmaceutical agents (agent for the prophylaxis or treatment for emotional disorders, an agent for the prophylaxis or treatment of depression accompanied by urinary frequency, urinary incontinence and/or irritable bowel syndrome, an agent for the prophylaxis or treatment of mood disorders of patients with urinary frequency and urinary incontinence, circadian rhythm controllers for the hypothalamic endocrine system and the like) of the present invention, they are, either directly or after having been mixed with suitable, pharmaceutically-acceptable carriers, for example, vehicles (e.g., starch, lactose, sucrose, calcium carbonate, calcium phosphate, etc.), binders (e.g., starch, arabic gum, carboxymethyl cellulose, hydroxypropyl cellulose, crystalline cellulose, alginic acid, gelatin, polyvinyl pyrrolidone, etc.), lubricants (e.g., stearic acid, magnesium stearate, calcium stearate, talc, etc.), disintegrators (e.g., calcium carboxymethyl cellulose, talc, etc.), diluents (e.g., water for injection, physiological saline, etc.) and optionally with additives (e.g., stabilizer, preservative, colorant, fragrance, dissolution aid, emulsifier, buffer, isotonic agent, etc.), etc., formulated into solid preparations such as powders, fine granules, granules, tablets, capsules, etc., or into liquid preparations such as injections, etc., by conventional methods for peroral or parenteral administration.

The pharmaceutical agent of the present invention may be in any solid forms of powder, granule, tablet, capsule, suppository, etc., and in any liquid forms of syrup, emulsion, injection, suspension, etc.

The pharmaceutical agent of the present invention can be produced by any conventional methods of, for example, blending, kneading, granulation, tableting, coating, sterilization, emulsification, etc., in accordance with the forms of the preparations to be produced. For the production of such pharmaceutical preparations, for example, referred to are the particular items in the general remarks for pharmaceutical preparations in the Japanese Pharmacopeia.

Preferably, various pharmaceutical agents of the present invention are prepared into various dosage forms suitable for oral administration. Specifically, the aforementioned dosage form is exemplified.

In the pharmaceutical agent of the present invention, the content of the NK-1 receptor antagonist or prodrug thereof is, though varying depending on the forms of the preparations, generally from about 0.01 to 100% by weight or so, preferably from about 0.1 to 50% by weight or so, more preferably from about 0.5 to 20% by weight or so, relative to the total weight of each preparation.

In the pharmaceutical agent of the present invention, the content of components other than the NK-1 receptor antagonist is, though varying depending on the forms of the preparations, generally from 0 to 99.9% by weight or so, preferably from about 10 to 99.9% by weight or so, more preferably from about 20 to 90% by weight or so, relative to the total weight of each preparation.

The dose of the pharmaceutical agent of the present invention varies, depending on the kind of the NK-1 receptor antagonist or pharmaceutically-acceptable salts thereof, the administration route, the condition and the age of patients, etc. For example, the dose for oral administration of the pharmaceutical agent to an adult patient suffering from depression is, in general, from about 0.005 to 50 mg/kg (body weight)/day, preferably from about 0.05 to 10 mg/kg (body weight)/day, more preferably from about 0.2 to 4 mg/kg (body weight)/day, in terms of the NK-1 receptor antagonist, which may be administered once a day or in one to three portions.

The agent for the prophylaxis or therapy of the present invention can be used in admixture with a suitable amount of a pharmaceutical active ingredient (combination drug) other than the NK-1 receptor antagonist, or in combination with a suitable amount thereof. Such active ingredients include, for example, drugs for central nervous systems (e.g., imipramine, etc.), anti-cholinergic drugs, $\alpha_1$-receptor-blocking drugs (e.g., tamsulosin, etc.), muscle relaxants (e.g., baclofen, etc.), potassium channel-opening drugs (e.g., nicorandil, etc.), calcium channel-blocking drugs (e.g., nifedipine, etc.), NK-2 receptor antagonist, acetylcholine esterase inhibitor (e.g., distigmine, etc.), etc.

Examples of the anti-cholinergic drug include atropine, scopolamine, homatropine, tropicamide, cyclopentolate, scopolamine butylbromide, propantheline bromide, methylbenactyzium bromide, mepenzolate bromide, flavoxate, pirenzepine, ipratropium bromide, trihexyphenidyl, oxybutynin, propiverine, darifenacin, tolterodine, temiverine, trospium chloride or a salt thereof (e.g., atropine sulfate, scopolamine hydrobromide, homatropine hydrobromide, cyclopentolate hydrochloride, flavoxate hydrochloride, pirenzepine hydrochloride, trihexyphenidyl hydrochloride, oxybutynin hydrochloride, tolterodine tartrate and the like) and the like. Preferably, oxybutynin, propiverine, darifenacin, tolterodine, temiverine, trospium chloride or a salt thereof (e.g., oxybutynin hydrochloride, tolterodine tartrate) are used.

Examples of the NK-2 receptor antagonist include GR94800, GR159897, MEN10627, MEN11420 (nepadutant), SR144190, SR48968 (saredutant) or a salt thereof and the like.

When the various pharmaceutical agents of the present invention and the above-mentioned combination drug are administered, the various pharmaceutical agents of the present invention and the combination drug may be administered at the same time, or the combination drug may be administered first and then the various pharmaceutical agents of the present invention may be administered, or the various pharmaceutical agents of the present invention may be administered first and thereafter the combination drug may be administered. In the administration with a time lag, the time difference varies depending on the active ingredient to be administered, dosage form and administration method. For example, when the combination drug is administered first, a method wherein the various pharmaceutical agents of the present invention are administered within 1 min - 3 days, preferably 10 min - 1 day, more preferably 15 min - 1 hr, after administration of the combination drug is exemplified. When the various pharmaceutical agents of the present invention are to be administered first, a method wherein the combination drug is administered within 1 min - 1 day, preferably 10 min - 6 hr, more preferably 15 min - 1 hr, after administration of the various pharmaceutical agents of the present invention is exemplified.

The present invention further provides a method for selecting an NK-1 receptor antagonist preferable for containing, as an active ingredient in the various pharmaceutical agents of the present invention, which is characterized by selecting an NK-1 receptor antagonist having one, preferably two, more preferably all three, of the following particular properties:

(i) having no serotonin uptake inhibitory effect,
(ii) being capable of migrating into the hypothalamus, and
(iii) having an inhibitory effect on micturition reflex of not less than 25% as compared to a control group.

The screening method is performed by measuring one of, preferably two of, more preferably all three of, the serotonin uptake inhibitory effect, capability of migration into the hypothalamus and inhibitory effect on micturition reflex, of the NK-1 receptor antagonist group.

The serotonin uptake inhibitory effect, capability of migration into the inhibitory effect on micturition reflex can be measured according to a method similar to the methods mentioned above.

The NK-1 receptor antagonist to be the target of screening may be any such as peptide, protein, non-peptide compound, synthesis compound, fermentation product and the like. These compounds may be novel compounds or known compounds. As a known NK-1 receptor antagonist, those described in the aforementioned patent publications

are used.

As the test animal, for example, normal or disease (e.g., depression) model of non-human mammals (e.g., mouse, rat, rabbit, sheep, pig, bovine, cat, dog, monkey and the like), more specifically depression rat, depression mouse, depression monkey and the like) and the like are used.

The NK-1 receptor antagonist or a prodrug thereof can be administered to a test animal according to a method known per se.

The NK-1 receptor antagonist and a prodrug thereof obtained by the screening method of the present invention show dramatically reduced side effects such as hypogonadism and the like observed in conventional anti-depressant, shows low toxicity and is safe. Therefore, a pharmaceutical composition containing an NK-1 receptor antagonist or a prodrug thereof obtained by the screening method of the present invention are useful as an agent for the prophylaxis or therapy of diseases such as an emotional disorder of mammals (e.g., mouse, rat, hamster, rabbit, cat, dog, bovine, sheep, monkey, human and the like) such as depression, depression accompanied by urinary frequency, urinary incontinence and/or irritable bowel syndrome, a mood disorder of patients with urinary incontinence and urinary frequency, abnormal circadian rhythm of the hypothalamic endocrine system, anxiety, circulatory psychosis, schizophrenia and the like.

A pharmaceutical agent containing an NK-1 receptor antagonist obtained by the screening method of the present invention, or a prodrug thereof can be produced and used in the same manner as in the aforementioned pharmaceutical agent of the present invention.

The anti-depressive action can be measured according to a method known per se, such as the method described in Science, pp. 1640-1645, vol. 281, 1998 or a similar method.

The present invention is explained in detail in the following by referring to examples. The present invention is not limited in any way by the examples and may be modified within the range that does not deviate from the scope of the present invention.

## Examples

### Example 1

| | | |
|---|---|---|
| (1) Compound No. 3 | 10 | mg |
| (2) Lactose | 60 | mg |
| (3) Corn starch | 35 | mg |
| (4) Hydroxypropylmethylcellulose | 3 | mg |
| (5) Magnesium stearate | 2 | mg |

A mixture of 10 mg of the compound No. 3 ((9R)-7-[3,5-bis(trifluoromethyl)benzyl]-6,7,8,9,10,11-hexahydro-9-methyl-5-(4-methylphenyl)-6,13-dioxo-13H-[1,4]diazocino[2,1-g][1,7]naphthyridine), 60 mg of lactose and 35 mg of corn starch were granulated using 10 wt% aqueous hydroxypropylmethylcellulose solution (0.03 ml, 3 mg as hydroxypropylmethylcellulose), dried at 40°C and passed through a sieve. The obtained granules were mixed with magnesium stearate (2 mg) and compressed. The obtained naked tablets were sugar-coated with an aqueous suspension of sucrose, dititanium oxide,.talc and gum arabic. The coated tablets were polished with bee wax to give coated tablets.

### Example 2

| | | |
|---|---|---|
| (1) Compound No. 3 | 10 | mg |
| (2) Lactose | 70 | mg |
| (3) Corn starch | 50 | mg |
| (4) Soluble starch | 7 | mg |
| (5) Magnesium stearate | 3 | mg |

The compound No. 3 (10 mg) and magnesium stearate (3 mg) were granulated using 0.07 ml of aqueous solution of soluble starch (7 mg as soluble starch), dried and admixed with lactose (70 mg) and corn starch (50 mg). The mixture was compressed to give tablets.

## Industrial Applicability

The various pharmaceutical preparations of the present invention containing an NK-1 receptor antagonist (particularly an NK-1 receptor antagonist having particular properties of (i) having no serotonin uptake inhibitory effect, ii) being capable of migrating into the hypothalamus, and iii) having an inhibitory effect on micturition reflex) shows reduced side effects such as hypogonadism and the like, and are useful as an agent for the prophylaxis or treatment of emotional disorders, an agent for the prophylaxis or treatment of depression accompanied by urinary frequency, urinary incontinence and/or irritable bowel syndrome, an agent for the prophylaxis or treatment of a mood disorder of patients with urinary incontinence and urinary frequency, and circadian rhythm controller for the hypothalamic endocrine system.

This application is based on patent application Nos. 2000-297086, 2000-391037, 2000-391088 and 2000-391106 filed in Japan, the contents of which are hereby incorporated by reference.

## Claims

1. An agent for the prophylaxis or treatment of an emotional disorder, which comprises an NK-1 receptor antagonist having at least one of the following properties:

   (i) having no serotonin uptake inhibitory effect,
   (ii) being capable of migrating into the hypothalamus,
   (iii) having an inhibitory effect on micturition reflex.

2. The agent of claim 1, wherein the emotional disorder is depression.

3. The agent of claim 2, wherein the depression accompanies urinary frequency, urinary incontinence and/or irritable bowel syndrome.

4. The agent of claim 1, wherein the emotional disorder is a mood disorder.

5. The agent of claim 1, wherein the emotional disorder accompanies abnormal circadian rhythm of the hypothalamic endocrine system.

6. The agent of claim 1, which is used for oral administration.

7. An agent for the prophylaxis or treatment of depression accompanied by urinary frequency, urinary incontinence and/or irritable bowel syndrome, which comprises an NK-1 receptor antagonist.

8. An agent for the prophylaxis or treatment of a mood disorder of patients with urinary frequency and urinary incontinence, which comprises an NK-1 receptor antagonist.

9. The agent of claim 7 or 8, which is used for oral administration.

10. A circadian rhythm controller for the hypothalamic endocrine system, which comprises an NK-1 receptor antagonist.

11. The circadian rhythm controller of claim 10, which is used for oral administration.

12. The circadian rhythm controller of claim 11, wherein the NK-1 receptor antagonist has at least one of the following properties:

    (i) having no serotonin uptake inhibitory effect,
    (ii) being capable of migrating into the hypothalamus,.
    (iii) having an inhibitory effect on micturition reflex.

13. A pharmaceutical composition for the prophylaxis or treatment of an emotional disorder, which comprises an NK-1 receptor antagonist having at least one of the following properties and a pharmaceutically acceptable carrier:

    (i) having no serotonin uptake inhibitory effect,

(ii) being capable of migrating into the hypothalamus,
(iii) having an inhibitory effect on micturition reflex.

**14.** The pharmaceutical composition of claim 13, wherein the emotional disorder is depression, depression accompanied by urinary frequency, urinary incontinence and/or irritable bowel syndrome, a mood disorder (particularly a mood disorder of patients with urinary frequency and urinary incontinence) or an emotional disorder accompanying abnormal circadian rhythm of the hypothalamic endocrine system.

**15.** A pharmaceutical composition for the prophylaxis or treatment of depression accompanied by urinary frequency, urinary incontinence and/or irritable bowel syndrome, which comprises an NK-1 receptor antagonist and a pharmaceutically acceptable carrier.

**16.** A pharmaceutical composition for the prophylaxis or treatment of a mood disorder of patients with urinary frequency and urinary incontinence, which comprises an NK-1 receptor antagonist and a pharmaceutically acceptable carrier.

**17.** A pharmaceutical composition for controlling circadian rhythm of the hypothalamic endocrine system, which comprises an NK-1 receptor antagonist and a pharmaceutically acceptable carrier.

**18.** The pharmaceutical composition of claim 17, wherein the NK-1 receptor antagonist has at least one of the following properties:

(i) having no serotonin uptake inhibitory effect,
(ii) being capable of migrating into the hypothalamus,
(iii) having an inhibitory effect on micturition reflex.

**19.** A method for the prophylaxis or treatment of an emotional disorder, which comprises administering an effective amount of an NK-1 receptor antagonist having at least one of the following properties to a patient:

(i) having no serotonin uptake inhibitory effect,
(ii) being capable of migrating into the hypothalamus,
(iii) having an inhibitory effect on micturition reflex.

**20.** The method of claim 19, wherein the emotional disorder is depression, depression accompanied by urinary frequency, urinary incontinence and/or irritable bowel syndrome, a mood disorder (particularly a mood disorder of patients with urinary frequency and urinary incontinence) or an emotional disorder accompanying abnormal circadian rhythm of the hypothalamic endocrine system.

**21.** A method for the prophylaxis or treatment of depression accompanied by urinary frequency, urinary incontinence and/or irritable bowel syndrome, which comprises administering an effective amount of an NK-1 receptor antagonist to a patient.

**22.** A method for the prophylaxis or treatment of a mood disorder of patients with urinary frequency and urinary incontinence, which comprises administering an effective amount of an NK-1 receptor antagonist to a patient.

**23.** A method for controlling circadian rhythm of the hypothalamic endocrine system, which comprises administering an effective amount of an NK-1 receptor antagonist to a patient.

**24.** The method of claim 23, wherein the NK-1 receptor antagonist has at least one of the following properties:

(i) having no serotonin uptake inhibitory effect,
(ii) being capable of migrating into the hypothalamus,
(iii) having an inhibitory effect on micturition reflex.

**25.** Use of an NK-1 receptor antagonist for the production of an agent for the prophylaxis or treatment of an emotional disorder, wherein the antagonist has at least one of the following properties:

(i) having no serotonin uptake inhibitory effect,
(ii) being capable of migrating into the hypothalamus,

(iii) having an inhibitory effect on micturition reflex.

**26.** The use of claim 25, wherein the emotional disorder is depression, depression accompanied by urinary frequency, urinary incontinence and/or irritable bowel syndrome, a mood disorder (particularly a mood disorder of patients with urinary frequency and urinary incontinence) or an emotional disorder accompanying abnormal circadian rhythm of the hypothalamic endocrine system.

**27.** Use of an NK-1 receptor antagonist for the production of an agent for the prophylaxis or treatment of depression accompanied by urinary frequency, urinary incontinence and/or irritable bowel syndrome.

**28.** Use of an NK-1 receptor antagonist for the production of an agent for the prophylaxis or treatment of a mood disorder of patients with urinary frequency and urinary incontinence.

**29.** Use of an NK-1 receptor antagonist for the production of a circadian rhythm controller for the hypothalamic endocrine system.

**30.** The use of claim 29, wherein the NK-1 receptor antagonist has at least one of the following properties:

(i) having no serotonin uptake inhibitory effect,
(ii) being capable of migrating into the hypothalamus,
(iii) having an inhibitory effect on micturition reflex.

**31.** A commercial package comprising the pharmaceutical composition of claim 13 and written matter associated therewith, the written matter stating that the pharmaceutical composition can or should be used for the prophylaxis or treatment of an emotional disorder.

**32.** The commercial package of claim 31, wherein the emotional disorder is depression, depression accompanied by urinary frequency, urinary incontinence and/or irritable bowel syndrome, a mood disorder (particularly a mood disorder of patients with urinary frequency and urinary incontinence) or an emotional disorder accompanying abnormal circadian rhythm of the hypothalamic endocrine system.

**33.** A commercial package comprising the pharmaceutical composition of claim 15 and written matter associated therewith, the written matter stating that the pharmaceutical composition can or should be used for the prophylaxis or treatment of depression accompanied by urinary frequency, urinary incontinence and/or irritable bowel syndrome.

**34.** A commercial package comprising the pharmaceutical composition of claim 16 and a written matter associated therewith, the written matter stating that the pharmaceutical composition can or should be used for the prophylaxis or treatment of a mood disorder of patients with urinary frequency and urinary incontinence.

**35.** A commercial package comprising the pharmaceutical composition of claim 17 and written matter associated therewith, the written matter stating that the pharmaceutical composition can or should be used for controlling circadian rhythm of the hypothalamic endocrine system.

**36.** A screening method of an NK-1 receptor antagonist, which comprises measuring an NK-1 receptor antagonist group for at least one action selected from the group consisting of (i) serotonin uptake inhibitory effect, (ii) capability of migration into the hypothalamus and (iii) an inhibitory effect on micturition reflex, and determining an NK-1 receptor antagonist having at least one of the following properties:

(i) having no serotonin uptake inhibitory effect,
(ii) being capable of migrating into the hypothalamus,
(iii) having an inhibitory effect on micturition reflex of not less than 25% as compared to a control group.

**37.** The agent of claim 1, wherein the NK-1 receptor antagonist is obtained by the screening method of claim 36.

**38.** The agent of claim 7, wherein the NK-1 receptor antagonist is obtained by the screening method of claim 36.

**39.** The agent of claim 8, wherein the NK-1 receptor antagonist is obtained by the screening method of claim 36.

**40.** The circadian rhythm controller of claim 10, wherein the NK-1 receptor antagonist is obtained by the screening method of claim 36.

**INTERNATIONAL SEARCH REPORT**

International application No.
PCT/JP01/08346

A. CLASSIFICATION OF SUBJECT MATTER
Int.Cl[7] A61K45/00, A61K31/4985, A61K31/551, A61K31/4375, A61K31/553, A61K31/4353, A61P43/00, A61P25/14, A61P13/02, A61P25/24, A61P1/00, A61P25/20, C07D471/14, C07D498/04
According to International Patent Classification (IPC) or to both national classification and IPC

B. FIELDS SEARCHED
Minimum documentation searched (classification system followed by classification symbols)
Int.Cl[7] A61K45/00, A61K31/4985, A61K31/551, A61K31/4375, A61K31/553, A61K31/4353, A61P43/00, A61P25/14, A61P13/02, A61P25/24, A61P1/00, A61P25/20, C07D471/14, C07D498/04

Documentation searched other than minimum documentation to the extent that such documents are included in the fields searched
Jitsuyo Shinan Koho 1922-1996 Toroku Jitsuyo Shinan Koho 1994-2001
Kokai Jitsuyo Shinan Koho 1971-2001 Jitsuyo Shinan Toroku Koho 1996-2001

Electronic data base consulted during the international search (name of data base and, where practicable, search terms used)
CA(STN),EMBASE(STN),MEDLINE(STN),BIOSIS(STN)

C. DOCUMENTS CONSIDERED TO BE RELEVANT

| Category* | Citation of document, with indication, where appropriate, of the relevant passages | Relevant to claim No. |
|---|---|---|
| Y | Kramer,M.S. et al., Distinct Mechanism for Antidepressant Activity by Blockade of Central Subsstance P Receptors, Science, (1998), Vol.281, pages 1640 to 1645 | 1-40 |
| Y | Gitlin M.J., Effects of depression and antidepressants on sexual functioning, Bull. Menninger Clin., (1995), Vol.59, No.2, pages 232 to 248 | 1-40 |
| Y | Nussdorfer G.G., Role of tachykinins inthe regulation of the hypothalamo-pituitary-adrebak axis, Peptides, (1998), Vol.19, No.5, pages 949 to 968 | 1-40 |
| Y | Carlo Alberto Maggi et atl., Tachykinin receptors and tachykinin receptor antagonists, J. Suton. Pharmacol., (1993), pages 23 to 93 | 1-40 |
| Y | EP 577590 A (Takeda Chemical Industries, Ltd.), 23 June, 1998 (23.06.98), Full text & JP 9-263585 A & US 5786352 A & US 6147071 A & CN 1140172 A | 1-40 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 10 December, 2001 (10.12.01) | 18 December, 2001 (18.12.01) |
| Name and mailing address of the ISA/ Japanese Patent Office | Authorized officer |
| Facsimile No. | Telephone No. |

Form PCT/ISA/210 (second sheet) (July 1992)

**INTERNATIONAL SEARCH REPORT**

International application No.
PCT/JP01/08346

C (Continuation). DOCUMENTS CONSIDERED TO BE RELEVANT

| Category* | Citation of document, with indication, where appropriate, of the relevant passages | Relevant to claim No. |
|---|---|---|
| Y | JP 10-109989 A (Takeda Chemical Industries, Ltd.), 28 April, 1998 (28.04.98), Full text (Family: none) | 1-40 |

Form PCT/ISA/210 (continuation of second sheet) (July 1992)